# EUROPEAN PATENT APPLICATION

(11) **EP 1 167 388 A1**
(43) Date of publication of application: **02.01.2002**
(21) Application number: 00907989.8
(22) Date of filing: 10.03.2000
(51) Int. Cl.: C07K 16/18, C12N 15/12, C07K 16/46, C12N 5/18, C12N 5/16, C12N 1/21, A61K 38/17, A61K 39/395, A61P 7/00

(54) **SINGLE-STRANDED FV INDUCING APOPTOSIS**

(30) Priority: 10.03.1999 JP 6355799
(71) Applicant: CHUGAI SEIYAKU KABUSHIKI KAISHA, Tokyo, 115-8543 (JP)
(72) Inventor: FUKUSHIMA, Naoshi, Gotemba-shi Shizuoka 412-8513 (JP); UNO, Shinsuke, Gotemba-shi Shizuoka 412-8513 (JP)
(74) Representative: HOFFMANN - EITLE
(86) International application number: JP0001458
(87) International publication number: WO0053634

(57) **Abstract**

This invention relates to novel single-chain Fvs capable of inducing apoptosis of nucleated blood cells having Integrin Associated Protein (IAP). The single-chain Fvs of the invention comprise an L chain comprising the L chain V region of the mouse monoclonal antibodies capable of inducing apoptosis of cells having human IAP, an H chain comprising the H chain V region of the mouse monoclonal antibodies capable of inducing apoptosis of cells having human IAP and a linker connecting them. The single-chain Fvs of the invention are useful as a therapeutic agent for blood dyscrasia such as leukemia.

SEQUENCE LISTING

<110> CHUGAI SEIYAKU KABUSHIKI KAISHA

<120> Single-stranded Fv inducing apoptosis

<130> FOP-391

<160> 26

<210> 1

<211> 27

<212> DNA

<213> Artificial Sequence

<220>

<223> PCR primer

<400> 1

ccatcctaat acgactcact atagggc 27

<210> 2

<211> 27

<212> DNA

<213> Artificial Sequence

<220>

<223> PCR primer

<400> 2

ggatcccggg tggatggtgg gaagatg 27

<210> 3

<211> 28

<212> DNA

<213> Artificial Sequence

<220>

<223> PCR primer

<400> 3

ggatcccggg ccagtggata gacagatg 28

<210> 4

<211> 26

<212> DNA

<213> Artificial Sequence

<220>

<223> PCR primer

<400> 4

ggatcccggg agtggataga ccgatg 26

<210> 5

<211> 394

<212> DNA

<213> Mus

<220>

<221> CDS

<222> (1)...(393)

<223> pGEM-M1L.1~57;signal peptide,58~394;mature peptide

<400> 5

atg aag ttg cct gtt agg ctg ttg gtg ctg atg ttc tgg att cct 45

Met Lys Leu Pro Val Arg Leu Leu Val Leu Met Phe Trp Ile Pro

                5                   10                  15

gcg tcc agc agt gat gtt gtg atg acc caa act cca ctc tcc ctg 90
Ala Ser Ser Ser Asp Val Val Met Thr Gln Thr Pro Leu Ser Leu
                20                  25                  30
cct gtc agt ctt gga gat caa gcc tcc atc tct tgc aga tct agt 135
Pro Val Ser Leu Gly Asp Gln Ala Ser Ile Ser Cys Arg Ser Ser
                35                  40                  45
cag agc ctt cta cac agt aaa gga aac acc tat tta caa tgg tac 180
Gln Ser Leu Leu His Ser Lys Gly Asn Thr Tyr Leu Gln Trp Tyr
                50                  55                  60
cta cag aag cca ggc cag tct cca aag ctc ctg atc tac aaa gtt 225
Leu Gln Lys Pro Gly Gln Ser Pro Lys Leu Leu Ile Tyr Lys Val
                65                  70                  75
tcc aac cga ttt tct ggg gtc cca gac agg ttc agt ggc agt gga 270
Ser Asn Arg Phe Ser Gly Val Pro Asp Arg Phe Ser Gly Ser Gly
                80                  85                  90
tca ggg aca gat ttc aca ctc aag atc agc aga gtg gag gct gag 315
Ser Gly Thr Asp Phe Thr Leu Lys Ile Ser Arg Val Glu Ala Glu
                95                  100                 105
gat ctg gga gtt tat ttc tgc tct caa agt aca cat gtt ccg tac 360
Asp Leu Gly Val Tyr Phe Cys Ser Gln Ser Thr His Val Pro Tyr
                110                 115                 120
acg tcc gga ggg ggg acc aag ctg gaa ata aaa c 394
Thr Ser Gly Gly Gly Thr Lys Leu Glu Ile Lys
                125                 130

<210> 6
<211> 409

<212> DNA

<213> Mus

<220>

<221> CDS

<222> (1)...(408)

<223> pGEM-M1H.1-57;signal peptide,58-409;mature peptide

<400> 6

atg gaa tgg agc tgg ata ttt ctc ttc ctc ctg tca gga act gca 45

Met Glu Trp Ser Trp Ile Phe Leu Phe Leu Leu Ser Gly Thr Ala

                5                   10                  15

ggt gtc cac tcc cag gtc cag ctg cag cag tct gga cct gac ctg 90

Gly Val His Ser Gln Val Gln Leu Gln Gln Ser Gly Pro Asp Leu

                20                  25                  30

gta aag cct ggg gct tca gtg aag atg tcc tgc aag gct tct gga 135

Val Lys Pro Gly Ala Ser Val Lys Met Ser Cys Lys Ala Ser Gly

                35                  40                  45

tac acc ttc gtt aac cat gtt atg cac tgg gtg aag cag aag cca 180

Tyr Thr Phe Val Asn His Val Met His Trp Val Lys Gln Lys Pro

                50                  55                  60

ggg cag ggc ctt gag tgg att gga tat att tat cct tac aat gat 225

Gly Gln Gly Leu Glu Trp Ile Gly Tyr Ile Tyr Pro Tyr Asn Asp

                65                  70                  75

ggt act aag tac aat gag aag ttc aag ggc aag gcc aca ctg act 270

Gly Thr Lys Tyr Asn Glu Lys Phe Lys Gly Lys Ala Thr Leu Thr

                80                  85                  90

tca gag aaa tcc tcc agc gca gcc tac atg gag ctc agc agc ctg 315

Ser Glu Lys Ser Ser Ser Ala Ala Tyr Met Glu Leu Ser Ser Leu

                95                  100                 105
gcc tct gag gac tct gcg gtc tac tac tgt gca aga ggg ggt tac 360
Ala Ser Glu Asp Ser Ala Val Tyr Tyr Cys Ala Arg Gly Gly Tyr
                110                 115                 120
tat agt tac gac gac tgg ggc caa ggc acc act ctc aca gtc tcc 405
Tyr Ser Tyr Asp Asp Trp Gly Gln Gly Thr Thr Leu Thr Val Ser
                125                 130                 135
tca g 409
Ser

<210> 7
<211> 394
<212> DNA
<213> Mus
<220>
<221> CDS
<222> (1)...(393)
<223> pGEM-M2L. 1~57;signal peptide, 58~394;mature peptide
<400> 7
atg aag ttg cct gtt agg ctg ttg gtg ctg atg ttc tgg att cct 45
Met Lys Leu Pro Val Arg Leu Leu Val Leu Met Phe Trp Ile Pro
                5                   10                  15
ggt tcc agc agt gat gtt gtg atg acc caa agt cca ctc tcc ctg 90
Gly Ser Ser Ser Asp Val Val Met Thr Gln Ser Pro Leu Ser Leu
                20                  25                  30
cct gtc agt ctt gga gat caa gcc tcc atc tct tgc aga tca agt 135
Pro Val Ser Leu Gly Asp Gln Ala Ser Ile Ser Cys Arg Ser Ser

35                    40                    45

cag agc ctt gtg cac agt aat gga aag acc tat tta cat tgg tac 180

Gln Ser Leu Val His Ser Asn Gly Lys Thr Tyr Leu His Trp Tyr

50                    55                    60

ctg cag aag cca ggc cag tct cca aaa ctc ctg atc tac aaa gtt 225

Leu Gln Lys Pro Gly Gln Ser Pro Lys Leu Leu Ile Tyr Lys Val

65                    70                    75

tcc aac cga ttt tct ggg gtc cca gac agg ttc agt ggc agt gga 270

Ser Asn Arg Phe Ser Gly Val Pro Asp Arg Phe Ser Gly Ser Gly

80                    85                    90

tca gtg aca gat ttc aca ctc atg atc agc aga gtg gag gct gag 315

Ser Val Thr Asp Phe Thr Leu Met Ile Ser Arg Val Glu Ala Glu

95                    100                    105

gat ctg gga gtt tat ttc tgc tct caa agt aca cat gtt ccg tac 360

Asp Leu Gly Val Tyr Phe Cys Ser Gln Ser Thr His Val Pro Tyr

110                    115                    120

acg ttc gga ggg ggg acc aag ctg gaa ata aaa c 394

Thr Phe Gly Gly Gly Thr Lys Leu Glu Ile Lys

125                    130

<210> 8

<211> 409

<212> DNA

<213> Mus

<220>

<221> CDS

<222> (1)...(408)

<223> pGEM-M2H. 1~57;signal peptide, 58~409;mature peptide
<400> 8
atg gaa tgg agc tgg ata ttt ctc ttc ctc ctg tca gga act gca 45
Met Glu Trp Ser Trp Ile Phe Leu Phe Leu Leu Ser Gly Thr Ala
                5                   10                  15
ggt gtc cac tcc cag gtc cag ctg cag cag tct gga cct gaa ctg 90
Gly Val His Ser Gln Val Gln Leu Gln Gln Ser Gly Pro Glu Leu
                20                  25                  30
gta aag cct ggg gct tca gtg aag atg tcc tgc aag gct tct gga 135
Val Lys Pro Gly Ala Ser Val Lys Met Ser Cys Lys Ala Ser Gly
                35                  40                  45
tac acc ttc gct aac cat gtt att cac tgg gtg aag cag aag cca 180
Tyr Thr Phe Ala Asn His Val Ile His Trp Val Lys Gln Lys Pro
                50                  55                  60
ggg cag ggc ctt gag tgg att gga tat att tat cct tac aat gat 225
Gly Gln Gly Leu Glu Trp Ile Gly Tyr Ile Tyr Pro Tyr Asn Asp
                65                  70                  75
ggt act aag tat aat gag aag ttc aag gac aag gcc act ctg act 270
Gly Thr Lys Tyr Asn Glu Lys Phe Lys Asp Lys Ala Thr Leu Thr
                80                  85                  90
tca gac aaa tcc tcc acc aca gcc tac atg gac ctc agc agc ctg 315
Ser Asp Lys Ser Ser Thr Thr Ala Tyr Met Asp Leu Ser Ser Leu
                95                  100                 105
gcc tct gag gac tct gcg gtc tat tac tgt gca aga ggg ggt tac 360
Ala Ser Glu Asp Ser Ala Val Tyr Tyr Cys Ala Arg Gly Gly Tyr
                110                 115                 120
tat act tac gac gac tgg ggc caa ggc acc act ctc aca gtc tcc 405

Tyr Thr Tyr Asp Asp Trp Gly Gln Gly Thr Thr Leu Thr Val Ser
                125                 130                 135
tca g 409
Ser

<210> 9
<211> 32
<212> DNA
<213> Artificial Sequence
<220>
<223> PCR primer
<400> 9
cccaagcttc caccatgaag ttgcctgtta gg 32

<210> 10
<211> 32
<212> DNA
<213> Artificial Sequence
<220>
<223> PCR primer
<400> 10
cccaagcttc caccatggaa tggagctgga ta 32

<210> 11
<211> 34
<212> DNA
<213> Artificial Sequence

<220>

<223> PCR primer

<400> 11

cgcggatcca ctcacgtttt atttccagct tggt 34

<210> 12

<211> 34

<212> DNA

<213> Artificial Sequence

<220>

<223> PCR primer

<400> 12

cgcggatcca ctcacctgag gagactgtga gagt 34

<210> 13

<211> 30

<212> DNA

<213> Artificial Sequence

<220>

<223> PCR primer

<400> 13

catgccatgg cgcaggtcca gctgcagcag 30

<210> 14

<211> 27

<212> DNA

<213> Artificial Sequence

<220>

<223> PCR primer

<400> 14

accaccacct gaggagactg tgagagt 27

<210> 15

<211> 27

<212> DNA

<213> Artificial Sequence

<220>

<223> PCR primer

<400> 15

gtctcctcag gtggtggtgg ttcgggt 27

<210> 16

<211> 27

<212> DNA

<213> Artificial Sequence

<220>

<223> PCR primer

<400> 16

cacaacatcc gatccgccac cacccga 27

<210> 17

<211> 27

<212> DNA

<213> Artificial Sequence

<220>

<223> PCR primer

<400> 17

ggcggatcgg atgttgtgat gacccaa 27

<210> 18

<211> 57

<212> DNA

<213> Artificial Sequence

<220>

<223> PCR primer

<400> 18

ccggaattct cattatttat cgtcatcgtc tttgtagtct tttatttcca gcttggt 57

<210> 19

<211> 45

<212> DNA

<213> Artificial Sequence

<220>

<223> Linker amino acid sequence and nucleotide sequence

<400> 19

ggt ggt ggt ggt tcg ggt ggt ggt ggt tcg ggt ggt ggc gga tcg 45

Gly Gly Gly Gly Ser Gly Gly Gly Gly Ser Gly Gly Gly Gly Ser

                5                   10                  15

<210> 20

<211> 828

<212> DNA

<213> Mus

<220>

<221>CDS

<222>(1)...(826)

<223> pscM1. MABL1-scFv

<400> 20

atg aaa tac cta ttg cct acg gca gcc gct gga ttg tta tta ctc 45

Met Lys Tyr Leu Leu Pro Thr Ala Ala Ala Gly Leu Leu Leu Leu

                5                   10                  15

gct gcc caa cca gcc atg gcg cag gtc cag ctg cag cag tct gga 90

Ala Ala Gln Pro Ala Met Ala Gln Val Gln Leu Gln Gln Ser Gly

                20                  25                  30

cct gac ctg gta aag cct ggg gct tca gtg aag atg tcc tgc aag 135

Pro Asp Leu Val Lys Pro Gly Ala Ser Val Lys Met Ser Cys Lys

                35                  40                  45

gct tct gga tac acc ttc gtt aac cat gtt atg cac tgg gtg aag 180

Ala Ser Gly Tyr Thr Phe Val Asn His Val Met His Trp Val Lys

                50                  55                  60

cag aag cca ggg cag ggc ctt gag tgg att gga tat att tat cct 225

Gln Lys Pro Gly Gln Gly Leu Glu Trp Ile Gly Tyr Ile Tyr Pro

                65                  70                  75

tac aat gat ggt act aag tac aat gag aag ttc aag ggc aag gcc 270

Tyr Asn Asp Gly Thr Lys Tyr Asn Glu Lys Phe Lys Gly Lys Ala

                80                  85                  90

aca ctg act tca gag aaa tcc tcc agc gca gcc tac atg gag ctc 315

Thr Leu Thr Ser Glu Lys Ser Ser Ser Ala Ala Tyr Met Glu Leu

                95                  100                 105
agc agc ctg gcc tct gag gac tct gcg gtc tac tac tgt gca aga 360
Ser Ser Leu Ala Ser Glu Asp Ser Ala Val Tyr Tyr Cys Ala Arg
                110                 115                 120
ggg ggt tac tat agt tac gac gac tgg ggc caa ggc acc act ctc 405
Gly Gly Tyr Tyr Ser Tyr Asp Asp Trp Gly Gln Gly Thr Thr Leu
                125                 130                 135
aca gtc tcc tca ggt ggt ggt ggt tcg ggt ggt ggt ggt tcg ggt 450
Thr Val Ser Ser Gly Gly Gly Gly Ser Gly Gly Gly Gly Ser Gly
                140                 145                 150
ggt ggc gga tcg gat gtt gtg atg acc caa act cca ctc tcc ctg 495
Gly Gly Gly Ser Asp Val Val Met Thr Gln Thr Pro Leu Ser Leu
                155                 160                 165
cct gtc agt ctt gga gat caa gcc tcc atc tct tgc aga tct agt 540
Pro Val Ser Leu Gly Asp Gln Ala Ser Ile Ser Cys Arg Ser Ser
                170                 175                 180
cag agc ctt cta cac agt aaa gga aac acc tat tta caa tgg tac 585
Gln Ser Leu Leu His Ser Lys Gly Asn Thr Tyr Leu Gln Trp Tyr
                185                 190                 195
cta cag aag cca ggc cag tct cca aag ctc ctg atc tac aaa gtt 630
Leu Gln Lys Pro Gly Gln Ser Pro Lys Leu Leu Ile Tyr Lys Val
                200                 205                 210
tcc aac cga ttt tct ggg gtc cca gac agg ttc agt ggc agt gga 675
Ser Asn Arg Phe Ser Gly Val Pro Asp Arg Phe Ser Gly Ser Gly
                215                 220                 225
tca ggg aca gat ttc aca ctc aag atc agc aga gtg gag gct gag 720
Ser Gly Thr Asp Phe Thr Leu Lys Ile Ser Arg Val Glu Ala Glu

                230                 235                 240
gat ctg gga gtt tat ttc tgc tct caa agt aca cat gtt ccg tac 765
Asp Leu Gly Val Tyr Phe Cys Ser Gln Ser Thr His Val Pro Tyr
                245                 250                 255
acg tcc gga ggg ggg acc aag ctg gaa ata aaa gac tac aaa gac 810
Thr Ser Gly Gly Gly Thr Lys Leu Glu Ile Lys Asp Tyr Lys Asp
                260                 265                 270
gat gac gat aaa taa tga 828
Asp Asp Asp Lys

<210> 21
<211> 31
<212> DNA
<213> Artificial Sequence
<220>
<223> PCR primer
<400> 21
acgcgtcgac tcccaggtcc agctgcagca g 31

<210> 22
<211> 18
<212> DNA
<213> Artificial Sequence
<220>
<223> PCR primer
<400> 22
gaaggtgtat ccagaagc 18

<210> 23

<211> 819

<212> DNA

<213> Mus

<220>

<221> CDS

<222>(1)...(813)

<223> pCHOM1. MABL1-scFv

<400> 23

atg gga tgg agc tgt atc atc ctc ttc ttg gta gca aca gct aca 45

Met Gly Trp Ser Cys Ile Ile Leu Phe Leu Val Ala Thr Ala Thr

                5                   10                  15

ggt gtc gac tcc cag gtc cag ctg cag cag tct gga cct gac ctg 90

Gly Val Asp Ser Gln Val Gln Leu Gln Gln Ser Gly Pro Asp Leu

                20                  25                  30

gta aag cct ggg gct tca gtg aag atg tcc tgc aag gct tct gga 135

Val Lys Pro Gly Ala Ser Val Lys Met Ser Cys Lys Ala Ser Gly

                35                  40                  45

tac acc ttc gtt aac cat gtt atg cac tgg gtg aag cag aag cca 180

Tyr Thr Phe Val Asn His Val Met His Trp Val Lys Gln Lys Pro

                50                  55                  60

ggg cag ggc ctt gag tgg att gga tat att tat cct tac aat gat 225

Gly Gln Gly Leu Glu Trp Ile Gly Tyr Ile Tyr Pro Tyr Asn Asp

                65                  70                  75

ggt act aag tac aat gag aag ttc aag ggc aag gcc aca ctg act 270

Gly Thr Lys Tyr Asn Glu Lys Phe Lys Gly Lys Ala Thr Leu Thr

                80                  85                  90
tca gag aaa tcc tcc agc gca gcc tac atg gag ctc agc agc ctg 315
Ser Glu Lys Ser Ser Ser Ala Ala Tyr Met Glu Leu Ser Ser Leu
                95                  100                 105
gcc tct gag gac tct gcg gtc tac tac tgt gca aga ggg ggt tac 360
Ala Ser Glu Asp Ser Ala Val Tyr Tyr Cys Ala Arg Gly Gly Tyr
                110                 115                 120
tat agt tac gac gac tgg ggc caa ggc acc act ctc aca gtc tcc 405
Tyr Ser Tyr Asp Asp Trp Gly Gln Gly Thr Thr Leu Thr Val Ser
                125                 130                 135
tca ggt ggt ggt ggt tcg ggt ggt ggt ggt tcg ggt ggt ggc gga 450
Ser Gly Gly Gly Gly Ser Gly Gly Gly Gly Ser Gly Gly Gly Gly
                140                 145                 150
tcg gat gtt gtg atg acc caa act cca ctc tcc ctg cct gtc agt 495
Ser Asp Val Val Met Thr Gln Thr Pro Leu Ser Leu Pro Val Ser
                155                 160                 165
ctt gga gat caa gcc tcc atc tct tgc aga tct agt cag agc ctt 540
Leu Gly Asp Gln Ala Ser Ile Ser Cys Arg Ser Ser Gln Ser Leu
                170                 175                 180
cta cac agt aaa gga aac acc tat tta caa tgg tac cta cag aag 585
Leu His Ser Lys Gly Asn Thr Tyr Leu Gln Trp Tyr Leu Gln Lys
                185                 190                 195
cca ggc cag tct cca aag ctc ctg atc tac aaa gtt tcc aac cga 630
Pro Gly Gln Ser Pro Lys Leu Leu Ile Tyr Lys Val Ser Asn Arg
                200                 205                 210
TTT TCT GGG GTC CCA GAC AGG TTC AGT GGC AGT GGA TCA GGG ACA 675
Phe Ser Gly Val Pro Asp Arg Phe Ser Gly Ser Gly Ser Gly Thr

                215                 220                 225
gat ttc aca ctc aag atc agc aga gtg gag gct gag gat ctg gga 720
Asp Phe Thr Leu Lys Ile Ser Arg Val Glu Ala Glu Asp Leu Gly
                230                 235                 240
gtt tat ttc tgc tct caa agt aca cat gtt ccg tac acg tcc gga 765
Val Tyr Phe Cys Ser Gln Ser Thr His Val Pro Tyr Thr Ser Gly
                245                 250                 255
ggg ggg acc aag ctg gaa ata aaa gac tac aaa gac gat gac gat 810
Gly Gly Thr Lys Leu Glu Ile Lys Asp Tyr Lys Asp Asp Asp Asp
                260                 265                 270
aaa taa tga 819
Lys

<210> 24
<211> 828
<212> DNA
<213> Mus
<220>
<221> CDS
<222> (1)...(822)
<223> pscM2. MABL2-scFv
<400> 24
atg aaa tac cta ttg cct acg gca gcc gct gga ttg tta tta ctc 45
Met Lys Tyr Leu Leu Pro Thr Ala Ala Ala Gly Leu Leu Leu Leu
                5                   10                  15
gct gcc caa cca gcc atg gcg cag gtc cag ctg cag cag tct gga 90
Ala Ala Gln Pro Ala Met Ala Gln Val Gln Leu Gln Gln Ser Gly

                20                  25                  30
cct gaa ctg gta aag cct ggg gct tca gtg aag atg tcc tgc aag 135
Pro Glu Leu Val Lys Pro Gly Ala Ser Val Lys Met Ser Cys Lys
                35                  40                  45
gct tct gga tac acc ttc gct aac cat gtt att cac tgg gtg aag 180
Ala Ser Gly Tyr Thr Phe Ala Asn His Val Ile His Trp Val Lys
                50                  55                  60
cag aag cca ggg cag ggc ctt gag tgg att gga tat att tat cct 225
Gln Lys Pro Gly Gln Gly Leu Glu Trp Ile Gly Tyr Ile Tyr Pro
                65                  70                  75
tac aat gat ggt act aag tat aat gag aag ttc aag gac aag gcc 270
Tyr Asn Asp Gly Thr Lys Tyr Asn Glu Lys Phe Lys Asp Lys Ala
                80                  85                  90
act ctg act tca gac aaa tcc tcc acc aca gcc tac atg gac ctc 315
Thr Leu Thr Ser Asp Lys Ser Ser Thr Thr Ala Tyr Met Asp Leu
                95                  100                 105
agc agc ctg gcc tct gag gac tct gcg gtc tat tac tgt gca aga 360
Ser Ser Leu Ala Ser Glu Asp Ser Ala Val Tyr Tyr Cys Ala Arg
                110                 115                 120
ggg ggt tac tat act tac gac gac tgg ggc caa ggc acc act ctc 405
Gly Gly Tyr Tyr Thr Tyr Asp Asp Trp Gly Gln Gly Thr Thr Leu
                125                 130                 135
aca gtc tcc tca ggt ggt ggt ggt tcg ggt ggt ggt ggt tcg ggt 450
Thr Val Ser Ser Gly Gly Gly Gly Ser Gly Gly Gly Gly Ser Gly
                140                 145                 150
ggt ggc gga tcg gat gtt gtg atg acc caa agt cca ctc tcc ctg 495
Gly Gly Gly Ser Asp Val Val Met Thr Gln Ser Pro Leu Ser Leu

                155                 160                 165
cct gtc agt ctt gga gat caa gcc tcc atc tct tgc aga tca agt 540
Pro Val Ser Leu Gly Asp Gln Ala Ser Ile Ser Cys Arg Ser Ser
                170                 175                 180
cag agc ctt gtg cac agt aat gga aag acc tat tta cat tgg tac 585
Gln Ser Leu Val His Ser Asn Gly Lys Thr Tyr Leu His Trp Tyr
                185                 190                 195
ctg cag aag cca ggc cag tct cca aaa ctc ctg atc tac aaa gtt 630
Leu Gln Lys Pro Gly Gln Ser Pro Lys Leu Leu Ile Tyr Lys Val
                200                 205                 210
tcc aac cga ttt tct ggg gtc cca gac agg ttc agt ggc agt gga 675
Ser Asn Arg Phe Ser Gly Val Pro Asp Arg Phe Ser Gly Ser Gly
                215                 220                 225
tca gtg aca gat ttc aca ctc atg atc agc aga gtg gag gct gag 720
Ser Val Thr Asp Phe Thr Leu Met Ile Ser Arg Val Glu Ala Glu
                230                 235                 240
gat ctg gga gtt tat ttc tgc tct caa agt aca cat gtt ccg tac 765
Asp Leu Gly Val Tyr Phe Cys Ser Gln Ser Thr His Val Pro Tyr
                245                 250                 255
acg ttc gga ggg ggg acc aag ctg gaa ata aaa gac tac aaa gac 810
Thr Phe Gly Gly Gly Thr Lys Leu Glu Ile Lys Asp Tyr Lys Asp
                260                 265                 270
gat gac gat aaa taa tga 828
Asp Asp Asp Lys

<210> 25
<211> 819

<212> DNA

<213> Mus

<220>

<221> CDS

<222> (1)...(813)

<223> pCHOM2. MABL2-scFv

<400> 25

atg gga tgg agc tgt atc atc ctc ttc ttg gta gca aca gct aca 45

Met Gly Trp Ser Cys Ile Ile Leu Phe Leu Val Ala Thr Ala Thr

                5                   10                  15

ggt gtc gac tcc cag gtc cag ctg cag cag tct gga cct gaa ctg 90

Gly Val Asp Ser Gln Val Gln Leu Gln Gln Ser Gly Pro Glu Leu

                20                  25                  30

gta aag cct ggg gct tca gtg aag atg tcc tgc aag gct tct gga 135

Val Lys Pro Gly Ala Ser Val Lys Met Ser Cys Lys Ala Ser Gly

                35                  40                  45

tac acc ttc gct aac cat gtt att cac tgg gtg aag cag aag cca 180

Tyr Thr Phe Ala Asn His Val Ile His Trp Val Lys Gln Lys Pro

                50                  55                  60

ggg cag ggc ctt gag tgg att gga tat att tat cct tac aat gat 225

Gly Gln Gly Leu Glu Trp Ile Gly Tyr Ile Tyr Pro Tyr Asn Asp

                65                  70                  75

ggt act aag tat aat gag aag ttc aag gac aag gcc act ctg act 270

Gly Thr Lys Tyr Asn Glu Lys Phe Lys Asp Lys Ala Thr Leu Thr

                80                  85                  90

tca gac aaa tcc tcc acc aca gcc tac atg gac ctc agc agc ctg 315

Ser Asp Lys Ser Ser Thr Thr Ala Tyr Met Asp Leu Ser Ser Leu

                95                  100                 105

gcc tct gag gac tct gcg gtc tat tac tgt gca aga ggg ggt tac 360

Ala Ser Glu Asp Ser Ala Val Tyr Tyr Cys Ala Arg Gly Gly Tyr

                110                 115                 120

tat act tac gac gac tgg ggc caa ggc acc act ctc aca gtc tcc 405

Tyr Thr Tyr Asp Asp Trp Gly Gln Gly Thr Thr Leu Thr Val Ser

                125                 130                 135

tca ggt ggt ggt ggt tcg ggt ggt ggt ggt tcg ggt ggt ggc gga 450

Ser Gly Gly Gly Gly Ser Gly Gly Gly Gly Ser Gly Gly Gly Gly

                140                 145                 150

tcg gat gtt gtg atg acc caa agt cca ctc tcc ctg cct gtc agt 495

Ser Asp Val Val Met Thr Gln Ser Pro Leu Ser Leu Pro Val Ser

                155                 160                 165

ctt gga gat caa gcc tcc atc tct tgc aga tca agt cag agc ctt 540

Leu Gly Asp Gln Ala Ser Ile Ser Cys Arg Ser Ser Gln Ser Leu

                170                 175                 180

gtg cac agt aat gga aag acc tat tta cat tgg tac ctg cag aag 585

Val His Ser Asn Gly Lys Thr Tyr Leu His Trp Tyr Leu Gln Lys

                185                 190                 195

cca ggc cag tct cca aaa ctc ctg atc tac aaa gtt tcc aac cga 630

Pro Gly Gln Ser Pro Lys Leu Leu Ile Tyr Lys Val Ser Asn Arg

                200                 205                 210

ttt tct ggg gtc cca gac agg ttc agt ggc agt gga tca gtg aca 675

Phe Ser Gly Val Pro Asp Arg Phe Ser Gly Ser Gly Ser Val Thr

                215                 220                 225

gat ttc aca ctc atg atc agc aga gtg gag gct gag gat ctg gga 720

Asp Phe Thr Leu Met Ile Ser Arg Val Glu Ala Glu Asp Leu Gly

                230                 235                 240
gtt tat ttc tgc tct caa agt aca cat gtt ccg tac acg ttc gga 765
Val Tyr Phe Cys Ser Gln Ser Thr His Val Pro Tyr Thr Phe Gly
                245                 250                 255
ggg ggg acc aag ctg gaa ata aaa gac tac aaa gac gat gac gat 810
Gly Gly Thr Lys Leu Glu Ile Lys Asp Tyr Lys Asp Asp Asp Asp
                260                 265                 270
aaa taa tga 819
Lys

<210> 26
<211> 456
<212> DNA
<213> Mus
<220>
<221> CDS
<222> (1)...(450)
<223> pCHO-shIAP. Soluble human IAP
<400> 26
atg tgg ccc ctg gta gcg gcg ctg ttg ctg ggc tcg gcg tgc tgc 45
Met Trp Pro Leu Val Ala Ala Leu Leu Leu Gly Ser Ala Cys Cys
                5                   10                  15
gga tca gct cag cta cta ttt aat aaa aca aaa tct gta gaa ttc 90
Gly Ser Ala Gln Leu Leu Phe Asn Lys Thr Lys Ser Val Glu Phe
                20                  25                  30
acg ttt tgt aat gac act gtc gtc att cca tgc ttt gtt act aat 135
Thr Phe Cys Asn Asp Thr Val Val Ile Pro Cys Phe Val Thr Asn

                35                  40                  45

atg gag gca caa aac act act gaa gta tac gta aag tgg aaa ttt 180

Met Glu Ala Gln Asn Thr Thr Glu Val Tyr Val Lys Trp Lys Phe

                50                  55                  60

aaa gga aga gat att tac acc ttt gat gga gct cta aac aag tcc 225

Lys Gly Arg Asp Ile Tyr Thr Phe Asp Gly Ala Leu Asn Lys Ser

                65                  70                  75

act gtc ccc act gac ttt agt agt gca aaa att gaa gtc tca caa 270

Thr Val Pro Thr Asp Phe Ser Ser Ala Lys Ile Glu Val Ser Gln

                80                  85                  90

tta cta aaa gga gat gcc tct ttg aag atg gat aag agt gat gct 315

Leu Leu Lys Gly Asp Ala Ser Leu Lys Met Asp Lys Ser Asp Ala

                95                  100                 105

gtc tca cac aca gga aac tac act tgt gaa gta aca gaa tta acc 360

Val Ser His Thr Gly Asn Tyr Thr Cys Glu Val Thr Glu Leu Thr

                110                 115                 120

aga gaa ggt gaa acg atc atc gag cta aaa tat cgt gtt gtt tca 405

Arg Glu Gly Glu Thr Ile Ile Glu Leu Lys Tyr Arg Val Val Ser

                125                 130                 135

tgg ttt tct cca aat gaa aat gac tac aag gac gac gat gac aag 450

Trp Phe Ser Pro Asn Glu Asn Asp Tyr Lys Asp Asp Asp Asp Lys

                140                 145                 150

tga tag 456

## Description

### TECHNICAL FIELD

This invention relates to novel single-chain Fvs capable of inducing apoptosis of nucleated blood cells having Integrin Associated Protein (IAP). The single-chain Fvs are useful as a therapeutic agent for blood dyscrasia such as leukemia, which will be described later.

### BACKGROUND ART

Granulocyte colony stimulating factors, such as recombinant granulocyte colony stimulating factor (rG-CSF), have been known as humoral factors that stimulate differentiation and proliferation of granulocytes. Reports based on in vivo experiments with mice have shown that administration of rG-CSF results in not only accelerated myelopoiesis in bone marrow but also notable extramedullary hemopoiesis in the spleen, and hence proliferation of all hemopoietic precursor cells, including hemopoietic stem cells, in the spleen. The mechanism of such extramedullary hemopoiesis in the spleen has been believed that stimulation by rG-CSF alters the hemopoietic microenvironment of the spleen and promotes the hemopoiesis supporting ability thereof, thus inducing hemopoiesis.

In order to elucidate the hemopoietic function in the spleen, the present inventors focused on stromal cells of the spleen following repeated administration of rG-CSF. The inventors have made efforts to examine how the hemopoietic function is promoted by rG-CSF via stromal cells, and have established a hemopoietic stromal cell line (CF-1 cells) from mouse spleen by repeated administration of rG-CSF. The inventors have studied the hemopoiesis supporting ability of the hemopoietic stromal cells and confirmed the colony stimulating activity in vitro and the hemopoietic stem cell supporting ability in vivo [Blood, 80, 1914 (1992)].

However, one cell line of the splenic stromal cells has been established (CF-1 cells) and its cytological characteristics have been studied, whereas specific antibodies which recognize the surface antigens of these cells have never been prepared, nor have their characteristics been elucidated yet in any way.

In light of the aforementioned findings relating to splenic stromal cells and the results of previous researches, the present inventors have earnestly made further research aiming at developing specific antibodies that can recognize the splenic stromal cells, made efforts to prepare monoclonal antibodies using the aforementioned splenic stromal cell line as a sensitizing antigen, and finally succeeded in obtaining novel monoclonal antibodies.

The inventors have further studied identities of the monoclonal antibodies obtained as above and found that the monoclonal antibodies are capable of inducing apoptosis of myeloid cells.

The inventors have also examined an antigen recognized by the antibody and found that the antigen is mouse Integrin Associated Protein (mouse IAP) (GeneBank, Accession Number Z25524).

The inventors studied the action of the antibodies using recombinant cells in which the mouse IAP gene had been introduced (Japanese Patent Application No. 09-67499).

In light of the aforementioned findings, the present inventors have succeeded in obtaining monoclonal antibodies of which the antigen is human Integrin Associated Protein (hereinafter referred to as human IAP; amino acid sequence and nucleotide sequence thereof are described in J. Cell Biol., 123, 485-496, 1993; see also Journal of Cell Science, 108, 3419-3425, 1995) and which are capable of inducing apoptosis of human nucleated blood cells having said antigen.

Further, the present inventors have succeeded in obtaining hybridomas which can produce novel monoclonal antibodies capable of inducing apoptosis of nucleated blood cells (myeloid cells and lymphocytes) having human Integrin Associated Protein (human IAP).

These hybridomas are hereinafter referred to as MABL-1 (FERM BP-6100) and MABL-2 (FERM BP-6101), and monoclonal antibodies produced by the hybridomas are also referred to as MABL-1 antibody and MABL-2 antibody, respectively.

### DISCLOSURE OF INVENTION

The inventors earnestly studied to utilize the aforementioned monoclonal antibodies derived from mice and having human IAP as antigen as a therapeutic agent for the later-mentioned blood dyscrasia.

An object of this invention is to provide antibodies which include a novel single-chain Fv capable of inducing apoptosis of nucleated blood cells having human IAP. The term "a single-chain Fv" used herein means a single chain polypeptide comprising an H chain V region and an L chain V region of the monoclonal antibodies.

Another object of the invention is to provide therapeutic agents against blood dyscrasia comprising the afore-mentioned substance which is capable of inducing apoptosis of nucleated blood cells having Integrin Associated Protein (IAP).

The present invention relates to single chain antibodies obtainable by reconstruction of the monoclonal antibodies derived from mice. More specifically, the invention relates to the reconstructed single-chain Fvs obtainable from the mouse monoclonal antibodies capable of inducing apoptosis of the nucleated blood cells having human IAP.

The present invention also relates to humanized antibodies of the reconstructed single-chain Fvs. Further, the invention relates to humanized monoclonal antibodies and fragments thereof which are producible from the foregoing humanized antibodies by the gene engineering approaches. The invention further provides human/mouse chimera antibodies, which are useful in the course of producing the reconstructed single-chain Fvs.

The present invention further relates to the process for genetically producing the reconstructed single-chain Fv of the mouse monoclonal antibodies, the humanized reconstructed single-chain Fv, the humanized monoclonal antibodies and fragments thereof and the chimera antibodies.

Specifically, the present invention relates to single-chain Fvs capable of inducing apoptosis of nucleated blood cells having human IAP, which comprise the L chain V region and the H chain V region of the mouse monoclonal antibodies (MABL-1 and MABL-2 antibodies) capable of inducing apoptosis of the nucleated blood cells having human IAP. The invention further relates to single-chain Fvs, wherein amino acid sequences of these V regions are partially modified.

Additionally, the present invention relates to the reconstructed humanized single-chain Fv, the reconstructed humanized monoclonal antibodies and the fragments of the humanized monoclonal antibodies, which are capable of inducing apoptosis of the nucleated blood cells having human IAP, and which are constructed of the reconstructed humanized L chain V region comprising a framework region (FR) and a CDR of the aforementioned mouse monoclonal antibodies, and the reconstructed humanized H chain V region comprising an FR and a CDR of the aforementioned mouse monoclonal antibodies. The invention also relates to the reconstructed humanized single-chain Fvs, the reconstructed humanized monoclonal antibodies and fragments thereof which have the same effect and in which amino acid sequences are partially modified.

Furthermore, the present invention relates to chimera antibodies capable of inducing apoptosis of nucleated blood cells having human IAP, which comprise an L chain comprising an L chain C region of human antibodies and an L chain V region of the aforementioned mouse monoclonal antibodies, and an H chain comprising an H chain C region of human antibodies and an H chain V region of the aforementioned mouse monoclonal antibodies.

The invention also relates to DNAs encoding the aforementioned antibodies, recombinant vectors comprising the DNAs and hosts transformed with the recombinant vectors.

The invention relates to a process for producing the reconstructed single-chain Fvs and the modified single-chain Fvs in which amino acid sequences are partially modified, which comprises culturing the above hosts and extracting the reconstructed single-chain Fvs from the culture thereof.

The invention further relates to a process for the production of the reconstructed humanized single-chain Fvs, the reconstructed humanized monoclonal antibodies and fragments thereof, and the reconstructed humanized single-chain Fvs, the reconstructed humanized monoclonal antibodies and fragments thereof in which amino acid sequences are partially modified, which are capable of inducing apoptosis of the nucleated blood cells having human IAP.

The invention further relates to a process for producing the chimera antibodies capable of inducing apoptosis of the nucleated blood cells having human IAP.

The present invention relates to therapeutic agents against blood dyscrasia comprising the substance as obtained above which is capable of inducing apoptosis of the nucleated blood cells having Integrin Associated Protein (IAP).

There is no method for producing the reconstructed single-chain Fvs that is applicable to the production of any specific antibodies and thus various means are needed to produce a reconstructed single-chain Fv sufficiently active to a specific antigen. Generally, a single chain antibody can be formed from a monoclonal antibody in the following manner, that is, by linking the H chain V region and the L chain V region derived from the monoclonal antibodies by using a linker. The resulting reconstructed single-chain Fvs contain variable regions of the parent antibodies and the complementarity determining region (CDR) thereof are preserved, and therefore the single-chain Fvs can be expected to bind to the antigen by the same specificity as that of the parent monoclonal antibodies.

The present invention also relates to CDRs derived from mammals other than mice, which correspond to the aforementioned mouse CDR, and a H chain V region and a L chain V region containing the CDRs. The CDRs and the H chain V regions and the L chain V regions include, for example, a CDR derived from human and a human H chain V region and a L chain V region containing the CDR, respectively.

The above-mentioned processes for producing the reconstructed single-chain Fvs are employed in the present invention.

### Cloning of a DNA encoding V region of mouse antibodies

In order to clone a DNA encoding the V region of the mouse monoclonal antibodies for human IAP, mRNAs are prepared from cells producing the mouse monoclonal antibody and converted to double strand cDNAs by a known method and the desired DNA is amplified from the cDNAs by polymerase chain reaction (PCR) method. As a source of mRNAs, a hybridoma producing a monoclonal antibody to human IAP should be prepared. Such hybridomas include MABL-1 (FERM BP-6100) and MABL-2 (FERM BP-6101). The monoclonal antibodies produced by the hybridomas, MABL-1 and MABL-2, are hereinafter referred to as MABL-1 antibody and MABL-2 antibody, respectively. A process for producing the hybridoma MABL-1 or MABL-2 will be described in Referential Example 1.
(1) Extraction of total RNA
   In the present invention, hybridoma cells are lysed with ISOGEN (Nippon Gene Inc.) and the resultant lysate is treated with isopropanol in order to extract total RNA. The processes which have already been used for the cloning of a gene of other protein can also be employed, for example, the process using the treatment with guanidine isothiocyanate followed by density-gradient centrifugation by cesium chloride (Chirgwin, J.M. et al., Biochemistry, 18, 5294-5299, 1979) and the process using the treatment with a surfactant in the presence of a ribonuclease inhibitor such as a vanadium complex followed by phenol treatment (Berger, S.L. et al., Biochemistry, 18, 5143-5149, 1979).
(2) Preparation of double-strand cDNA
   For the preparation of single strand DNAs from the total RNA prepared as above, the total RNA as a template is treated with a reverse transcriptase using oligo(dT) as a primer which is complementary to the poly A chain located at 3'-end of the RNA and the single strand DNA (cDNA) complementary to the total RNA can be synthesized (Larrik, J.W. et al., Bio/Technology, 7, 934-938, 1989). At that time, random primers may also be used.
(3) Amplification of V region of mouse antibody by polymerase chain reaction (PCR)
   The V region of the mouse antibody is specifically amplified from the cDNAs using the polymerase chain reaction (PCR). For the amplification of the V region of the mouse antibody, primers described in Jones, S.T. et al., Bio/Technology, 9, 88-89, 1991 may be employed. In order to select primers to be used for cloning the mouse monoclonal antibody produced by the hybridoma, MABL-1 or MABL-2, the typing of both H and L chains should be carried out.

The typing using ITOTYPING KIT (STRATAGENE Inc.) reveals that the MABL-1 antibody has a κ type L chain and a γ1 type H chain and that the MABL-2 antibody has a κ type L chain and a γ2a type H chain. The typing will be described in Referential Example 2.

Oligonucleotide primers of SEQ ID No.: 1 and SEQ ID No.: 2 are used as 5'-end and 3'-end primers, respectively, in order to amplify the L chain V region of the MABL-1 antibody by means of the polymerase chain reaction (PCR). The oligonucleotide primers of SEQ ID No.: 1 and SEQ ID No.: 2 are used as 5'-end and 3'-end primers, respectively, in order to amplify the L chain V region of the MABL-2 antibody.

The oligonucleotide primers of of SEQ ID No.: 1 and SEQ ID No.: 3 are used as 5'-end and 3'-end primers, respectively, in order to amplify the H chain V region of the MABL-1 antibody. The oligonucleotide primers of SEQ ID No.: 1 and SEQ ID No.: 4 are used as 5'-end and 3'-end primers, respectively, in order to amplify the H chain V region of the MABL-2 antibody.

In the present Examples, the 5'-primers which contain a sequence "GANTC" providing the restriction enzyme Hinf I digestion site at the neighborhood of 5'-terminal thereof are used and the 3'-primers which contain a nucleotide sequence "CCCGGG" providing the XmaI digestion site at the neighborhood of 5'-terminal thereof are used. Other restriction enzyme digestion sites may also be used as long as they are used for the purpose of subcloning a desired DNA fragment into a cloning vector.

The amplified product is isolated and purified using a low-melting temperature agarose or a column (PCR products purification kit (e.g. QIAGEN) or a DNA purification kit (e.g. GENECLEAN II) to obtain a desired DNA fragment encoding the variable region. A plasmid containing the DNA fragment encoding the desired variable region of the mouse monoclonal antibody is obtainable by linking the DNA fragment to a suitable cloning vector such as pGEM-T Easy.

Sequencing of cloned DNAs can be carried out by any conventional method, for example, an automatic DNA sequencer (Applied Biosystems Inc.).

The cloning and the sequencing of the desired DNAs will concretely be described in Examples 1 and 2.

### Complementarity determining region (CDR)

Each pair of the V regions of L and H chains forms an antigen binding site. The variable regions of the L and H chains link to comparatively conserved four framework regions with commonality and three hypervariable regions or complementarity determining regions (CDR) (Kabat, E.A. et al., "Sequences of Protein of Immunological Interest", US Dept. Health and Human Services, 1983).

Major portions in the four framework regions (FRs) form β-sheet structures and thus three CDRs form a loop. CDRs may form a part of the β-sheet structure in a certain case. The three CDRs are mutually held at the structurally closed position and contribute to the formation of the antigen binding site together with the three CDRs in the region forming a pair.

These CDRs can be found out by comparing the amino acid sequence of V region of the obtained antibody with known amino acid sequences of V regions of known antibodies according to the empirical rule in Kabat, E.A. et al., "Sequences of Protein of Immunological Interest". This will concretely be illustrated in Example 3.

### Preparation of chimera antibody

Prior to designing a single-chain Fv reconstructed from an antibody for human IAP, it should be confirmed that the employed CDRs actually form an antigen binding site. For this purpose, a chimera antigen is prepared. The amino acid sequences which are assumed from nucleotide sequences of the cloned DNAs of the monoclonal MABL-1 and MABL-2 antibodies described in Example 1 are compared with an amino acid sequence of the V region of the known mouse monoclonal antibody.

Cloning of a DNA fragment encoding V regions of L and H chains of the monoclonal antibody enables preparation of a chimera MABL-1 antibody or a chimera MABL-2 antibody by linking the resultant DNA encoding the mouse V region to a DNA encoding constant region of human antibody.

A basic method for preparing a chimera antibody comprises linking a mouse leader sequence and a sequence of V region existing in the cloned cDNA to a sequence coding the C region of a human antibody existing in an expression vector for mammal cells. The C region of the aforementioned human antibody may be any one of human L chain C regions and human H chain C regions, for example, human L chain Cκ, H chain γ-1 C and γ-4 C regions.

For the preparation of the chimera antibody, two expression vectors are prepared; i.e., an expression vector comprising a DNA encoding the mouse L chain V region and the human L chain C region under the control of an expression regulation region such as an enhancer/promoter system, and an expression vector comprising a DNA encoding the mouse H chain V region and the human H chain C region under the control of an expression regulation region such as an enhancer/promoter system. Then, a host cell such as a mammalian cell is co-transformed with these expression vectors and the transformed cell is cultured in vitro or in vivo to prepare the chimera antibody (e.g., WO91-16928).

Alternatively, a DNA encoding the mouse L chain V region and the human L chain C region and a DNA encoding the mouse H chain V region and the human H chain C region are introduced into a single expression vector, a host cell is transformed with the vector and the transformed host is cultured in vitro or in vivo in order to produce the desired chimera antibody.

The preparation of the chimera antibody will be described in Example 4.

A cDNA encoding a leader region and a V region of the L chain of the MABL-1 or MABL-2 antibody is subcloned by the PCR method and linked to an expression vector containing a genome DNA encoding a human genomic L chain C region.

A cDNA encoding an H chain leader region and a V region of the γ1 type of MABL-1 or MABL-2 antibody is subcloned by the PCR method and linked to an expression vector containing a genome DNA encoding a human genomic L chain Cκ region.

Specifically designed PCR primers are employed to provide suitable nucleotide sequences at 5'-end and 3'-end of the cDNAs encoding the V regions of the MABL-1 and MABL-2 antibodies so that the cDNAs may be readily inserted into an expression vector and appropriately function in the expression vector (e.g., transcription efficiency is increased by inserting Kozak sequence according to the invention). The V regions of the MABL-1 and MABL-2 antibodies obtained by amplifying by PCR using these primers are inserted into HEF expression vector containing the desired human C region (see WO92-19759). The vector is suitable for a transient expression or a stable expression of genetically modified antibodies in various mammalian cell lines.

The chimera MABL-1 and MABL-2 antibodies demonstrate an activity to bind to cells having human IAP. This confirms that correct mouse V regions have been cloned and that their sequences have been determined.

### Reconstructed single-chain Fv

For the production of a reconstructed single-chain Fv for cells having human IAP, the H chain V region and the L chain V region of the monoclonal antibody to human IAP are connected via a linker, preferably a peptide linker. A peptide linker includes any single chain linkers comprising 12 to 19 amino acids, for example, a peptide fragment described in SEQ ID No.: 19.

Concrete amino acid sequences of the reconstructed single-chain Fv are exemplified in SEQ ID Nos.: 20, 23, 24 and 25. In the invention, the single-chain Fvs having the amino acid sequences are referred to as MABL1-scFV and MABL2-scFv, which will be illustrated in Example 5.

The reconstructed single-chain Fvs of the invention are obtainable in the following manner; the DNA encoding the H chain V region of the MABL-1 or MABL-2 antibody and the DNA encoding the L chain V region of the MABL-1 or MABL-2 antibody, which are illustrated hereinabove, are employed as templates and a DNA encoding the desired amino acid sequence within these sequences is amplified by PCR method using a pair of primers which define both ends thereof.

A process for producing the reconstructed single-chain Fv comprising the H chain V region and the L chain V region will be concretely described in Example 5.

The antigen-binding activity of the reconstructed single-chain Fv can be evaluated in terms of the binding-inhibitory ability of the mouse MABL-1 and MABL-2 antibodies to human IAP as an index. Actually, the concentration-dependent inhibition of the mouse MABL-2 antibody to human IAP antigen is observed.

Preferably, an amino acid sequence of the aforementioned V regions may partially be modified in order to produce a reconstructed single-chain Fv which is sufficiently active for a specific antigen, if necessary.

The reconstructed single-chain Fv according to the present invention can be humanized by using conventional techniques (e.g. Sato, K. et al., Cancer Res., 53, 851-856 (1993)). Once a DNA encoding a humanized Fv is prepared, a humanized single-chain Fv, a fragment of the humanized single-chain Fv, a humanized monoclonal antibody and a fragment of the humanized monoclonal antibody can readily be produced according to conventional methods. Preferably, amino acid sequences of the V regions thereof are partially modified, if necessary.

As mentioned above, when the objective DNAs encoding the reconstructed single-chain Fv, the reconstructed humanized single-chain Fv, the humanized monoclonal antibodies and fragments thereof are prepared, the expression vectors containing them and hosts transformed with the vectors can be obtained according to conventional methods. Further, the hosts can be cultured according to a conventional method to produce the reconstructed single-chain Fv, the reconstructed humanized single-chain Fv, the humanized monoclonal antibodies and fragments thereof. These can be isolated from cells or a medium and can be purified uniformly, for which any isolation and purification method conventionally used for proteins may be employed without limitation thereto. The chimera antibodies or the humanized antibodies can be isolated and purified by suitable selection or combination of the methods, for example, various chromatographs, ultrafiltration, salting-out and dialysis.

For the production of the reconstructed single-chain Fv, the humanized single-chain Fv and the humanized monoclonal antibodies and fragments thereof against cells having human IAP according to the present invention, any expression systems can be employed, for example, eukaryotic cells such as an animal cell, e.g., an established mammalian cell line, filamentous fungi and yeast, and prokaryotic cells such as a bacterial cell, e.g., E. coli. Preferably, the chimera antibody or the reconstructed antibody of the invention is expressed in a mammal cell, for example COS7 cell or CHO cell.

In these cases, conventional promoters useful for the expression in a mammal cell can be used. For example, human cytomegalovirus (HCMV) immediate early promoter is preferably used. Expression vectors containing the HCMV promoter include HCMV-VH-HCγ 1, HCMV-VL-HCK and the like which are derived from pSV2neo (WO92-19759).

Other promoters for gene expression in a mammal cell that may be used in the invention include virus promoters derived form retrovirus, polyoma virus, adenovirus and simian virus 40 (SV40) and promoters derived from mammals such as human polypeptide-chain elongation factor-1α (HEF-1α). SV40 promoter can easily be used according to the method of Mulligan, R.C., et al. (Nature 277, 108-114 (1979)) and HEF-1α promoter can also be used according to the methods of Mizushima, S. et al. (Nucleic Acids Research, 18, 5322 (1990)).

Replication origin (ori) that can be used in the invention includes ori derived from SV40, polyoma virus, adenovirus, bovine papilloma virus (BPV) and the like. For the purpose of amplifying the gene copy number in the host cell system and the like, an expression vector may contain phosphotransferase APH (3') II or I (neo) gene, thymidine kinase (TK) gene, E. coli xanthine-guanine phosphoribosyl transferase (Ecogpt) gene or dihydrofolate reductase (DHFR) gene.

Reconstructed single chain Fvs generally have a superior mobility to tissues or tumors over whole IgG. Therefore, it is expected that MABL2-scFv constructed according to the invention can be used as a therapeutic agent for blood dyscrasia such as leukemia. It is further expected that MABL2-scFv can be used as a contrast agent by RI-labeling and that its effect can be enhanced by attaching to a RI-compound or a toxin.

### EXPLANATION OF DRAWINGS

Figure 1 shows the result of flow cytometry, illustrating that human IgG antibody does not bind to L1210 cells expressing human IAP (hIAP/L1210).

Figure 2 shows the result of flow cytometry, illustrating that the chimera MABL-1 antibody specifically binds to L1210 cells expressing human IAP (hIAP/L1210).

Figure 3 shows the result of flow cytometry, illustrating that the chimera MABL-2 antibody specifically binds to L1210 cells expressing human IAP (hIAP/L1210).

Figure 4 schematically illustrates the process for producing the single-chain Fv according to the present invention.

Figure 5 illustrates a structure of an expression plasmid which can be used to express a DNA encoding the single-chain Fv of the invention in E. coli.

Figure 6 illustrates a structure of the expression plasmid which is used to express a DNA encoding the single-chain Fv of the invention in mammal cells.

Figure 7 shows a photograph showing the result of western blotting in Example 5.4. From the left, a molecular weight marker (which indicates 97.4, 66, 45, 31, 21.5 and 14.5 kDa from the top), the cultured supernatant of pCHO1-introduced COS7 cells and the cultured supernatant of pCHOM2-introduced COS7 cells. The figure shows that the reconstructed single-chain Fv of the MABL-2 antibody (arrow) is contained in the cultured supernatant of the pCHOM2-introduced cells.

Figure 8 shows the result of flow cytometry, illustrating that an antibody in the cultured supernatant of pCHO1/COS7 cell as a control does not bind to pCOS1/L1210 cell as a control.

Figure 9 shows the result of flow cytometry, which illustrates that an antibody in the cultured supernatant of MABL2-scFv/COS7 cells does not bind to pCOS1/L1210 cells as a control.

Figure 10 shows the result of flow cytometry, illustrating that an antibody in the cultured supernatant of pCHO/COS7 cells as a control does not bind to hIAP/L1210 cells.

Figure 11 shows the result of flow cytometry, illustrating that an antibody in the cultured supernatant of MABL2-scFv/COS7 cells specifically binds to hIAP/L1210 cells.

Figure 12 shows the result of the competitive ELISA in Example 5.6, wherein the binding activity of the single-chain Fv of the invention (MABL2-scFv) to the antigen is demonstrated in terms of the inhibition of binding of the mouse monoclonal antibody MABL-2 to the antigen as an index, in comparison with the cultured supernatant of pCHO1/COS7 cells as a control.

Figure 13 shows the results of the apoptosis induction in Example 5.7, illustrating that the antibody in the cultured supernatant of pCHO1/COS7 cells as a control does not induce the apoptosis of pCOS1/L1210 cells as a control.

Figure 14 shows the results of the apoptosis induction in Example 5.7, illustrating that the antibody in the cultured supernatant of MABL2-scFv/COS7 cells does not induce apoptosis of pCOS1/L1210 cells as a control.

Figure 15 shows the results of the apoptosis induction in Example 5.7, illustrating that the antibody in the cultured supernatant of pCHO1/COS7 cells as a control does not induce apoptosis of hIAP/L1210 cells.

Figure 16 shows the results of the apoptosis induction in Example 5.7, illustrating that the antibody in the cultured supernatant of MABL2-scFv/COS7 cells specifically induces apoptosis of hIAP/L1210 cells.

Figure 17 shows the results of the apoptosis induction in Example 5.7, illustrating that the antibody in the cultured supernatant of pCHO1/COS7 cells as a control does not induce apoptosis of CCRF-CEM cells (50% final concentration).

Figure 18 shows the results of the apoptosis induction in Example 5.7, illustrating that the antibody in the cultured supernatant of MABL2-scFv/COS7 cells specifically induces apoptosis of CCRF-CEM cells (50% final concentration).

The present invention will concretely be illustrated in reference to the following examples, which in no way limit the scope of the invention.

### EXAMPLE

### Referential Example 1 (Preparation of hybridoma)

The cells highly expressing human Integrin Associated Protein (IAP) in L1210 cells, which are leukemia cell line derived from a DBA mouse (ATCC No. CCL-219; J. Natl. Cancer Inst. 10: 179-192, 1949), were prepared as described below and used as a sensitizing antibody.

The human IAP gene was amplified by PCR using cDNA prepared from mRNA of HL-60 cell line (CLONTECH Inc.) as a template.

This PCR product was introduced into a cloning vector, pGEM-T vector (Promega Inc.) and E. coli JM109 (Takara Inc.) was transformed with the resulting vector. A nucleotide sequence of the insert DNA was confirmed using a DNA sequencer (373 DNA Sequencer, ABI Inc.) and then the insert DNA was recombined with an expression vector pCOS1.

The expression vector pCOS1, which is a derivative of pEF-BOS (Nucleic Acids Research, 18, 5322, 1990), employs human elongation factor-1α as a promoter/enhancer and incorporates the neomycin resistant gene. This expression vector with human IAP incorporated was transfected to L1210 cell line with DMRIE-C (GIBCO-BRL). The L1210 cells were selected using Geneticin (the final concentration: 1 mg/ml, GIBCO-BRL) and cloned by the limiting dilution method. For the resulting clones, an expression of the antigen, human IAP, was analyzed using the anti-CD47 antibody recognizing human IAP (PharMingen) and a clone highly expressing the antigen was selected as an antigen-sensitizing cell.

Cell fusion between splenic cells of DBA/2 mouse (Japan Charles River Reproduction Inc.) which had been immunized with the aforementioned cells and mouse myeloma cell line P3-U1 (Current Topics in Micro-biology and Immunology, 81, 1-7 (1978)) was carried out according to a conventional method using polyethylene glycol (Clin. Exp. Immunol., 42, 458-462 (1980)). The DBA/2 mouse was the same strain as the L1210 cells.

Screening was performed using an activity of specifically recognizing human IAP as an indicator and two hybridomas were established. These have been designated as MABL-1 and MABL-2 and were internationally deposited as FERM BP-6100 and FERM BP-6101 on September 11, 1997 with the National Institute of Bioscience and Human Technology, Agency of Industrial Science and Technology, Minister of International Trade and Indusry of 1-3 Higasi 1-chome, Tsukuba-shi, Ibaraki-ken, Japan, as an authorized depository for microorganisms.

### Referential Example 2 (Subclass identification of MABL-1 and MABL-2 antibodies)

In order to identify the subclasses of the MABL-1 and MABL-2 antibodies as obtained above, 500 µl each of the MABL-1 and MABL-2 antibodies prepared at a level of 100 ng/ml was spotted on the Isotyping Kit (STRATAGENE). Consequently, it was revealed that the MABL-1 antibody is IgG1, κ type and the MABL-2 antibody is IgG2a, κ type.

### Example 1 (Cloning of DNAs encoding V region of mouse monoclonal antibodies to human IAP)

DNAs encoding variable regions of the mouse monoclonal antibodies, MABL-1 and MABL-2, to human IAP were cloned as follows.

### 1.1 Preparation of messenger RNA (mRNA)

mRNAs were prepared from the hybridomas MABL-1 and MABL-2 using the mRNA Purification Kit (Pharmacia Biotech).

### 1.2 Synthesis of double strand cDNA

Double strand cDNA was synthesized from about 1 µg of the mRNA using Marathon cDNA Amplification Kit (CLONTECH) and an adapter was linked thereto.

### 1.3 Amplification of genes encoding variable regions of an antibody by PCR

PCR was carried out using the Thermal Cycler (PERKIN ELMER).

### (1) Amplification of a gene coding L chain V region of MABL-1

Primers used for the PCR method are Adapter Primer-1 (CLONTECH) shown in SEQ ID No.: 1 which hybridizes to a partial sequence of the adapter and MKC (Mouse Kappa Constant) primer (Bio/Technology, 9, 88-89, 1991) shown in SEQ ID No.: 2 which hybridizes to the mouse kappa type L chain V region.

50 µl of the PCR solution contains 5 µl of 10 × PCR Buffer II, 2 mM.MgCl₂, 0.16 mM dNTPs (dATP, dGTP, dCTP and dTTP), 2.5 units of a DNA polymerase, AmpliTaq Gold (PERKIN ELMER), 0.2 µM of the adapter primer of SEQ ID No.: 1, 0.2 µM of the MKC of SEQ ID No.: 2 and 0.1 µg of the double strand cDNA derived from MABL-1. The solution was preheated at 94°C of the initial temperature for 9 minutes and then heated at 94°C for 1 minute, at 60°C for 1 minute and at 72°C for 1 minute 20 seconds in this order. This temperature cycle was repeated 35 times and then the reaction mixture was further heated at 72°C for 10 minutes.

### (2) Amplification of cDNA encoding H chain V region of MABL-1

The Adapter Primer-1 shown in SEQ ID No.: 1 and MHC-γ1 (Mouse Heavy Constant) primer (Bio/Technology, 9, 88-89, 1991) shown in SEQ ID No.: 3 were used as primers for PCR.

The amplification of cDNA was performed according to the method of the amplification of the L chain V region gene, which was described in Example 1.3-(1), except for using 0.2 µM of the MHC-γ1 primer instead of 0.2 µM of the MKC primer.

### (3) Amplification of cDNA encoding L chain V region of MABL-2

The Adapter Primer-1 of SEQ ID No.: 1 and the MKC primer of SEQ ID No.: 2 were used as primers for PCR.

The amplification of cDNA was carried out according to the method of the amplification of the L chain V region gene of MABL-1 which was described in Example 1.3-(1), except for using 0.1 µg of the double strand cDNA derived from MABL-2 instead of 0.1 µg of the double strand cDNA from MABL-1.

### (4) Amplification of cDNA encoding H chain V region of MABL-2

The Adapter Primer-1 of SEQ ID No.: 1 and MHC-γ2a primer (Bio/Technology, 9, 88-89, 1991) shown in SEQ ID No.: 4 were used as primers for PCR.

The amplification of cDNA was performed according to the method of the amplification of the L chain V region gene, which was described in Example 1.3-(3), except for using 0.2 µM of the MHC-γ2a primer instead of 0.2 µM of the MKC primer.

### 1.4 Purification of PCR products

The DNA fragment amplified by PCR as described above was purified using the QIAquick PCR Purification Kit (QIAGEN) and dissolved in 10 mM Tris-HCl (pH 8.0) containing 1 mM EDTA.

### 1.5 Ligation and Transformation

About 140 ng of the DNA fragment comprising the gene coding the mouse kappa type L chain V region derived from MABL-1 as prepared above was ligated with 50 ng of pGEM-T Easy vector (Promega) in the reaction buffer comprising 30 mM Tris-HCl (pH 7.8), 10 mM MgCl₂, 10 mM dithiothreitol, 1 mM ATP and 3 units of T4 DNA Ligase (Promega) at 15°C for 3 hours.

Then, 1 µl of the reaction mixture was added to 50 µl of E. coli DH5α competent cells (Toyobo Inc.) and the cells were stored on ice for 30 minutes, incubated at 42°C for 1 minute and stored on ice for 2 minutes again. 100 µl of SOC medium (GIBCO BRL) was added and the cells were plated on LB (Molecular Cloning: A Laboratory Manual, Sambrook et al., Cold Spring Harbor Laboratory Press, 1989) agar medium containing 100 µg/ml of ampicillin (SIGMA) and cultured at 37°C overnight to obtain the transformant of E. coli.

The transformant was cultured in 3 ml of LB medium containing 50 µg/ml of ampicillin at 37°C overnight and the plasmid DNA was prepared from the culture using the QIAprep Spin Miniprep Kit (QIAGEN).

The resulting plasmid comprising the gene coding the mouse kappa type L chain V region derived from the hybridoma MABL-1 was designated as pGEM-M1L.

According to the same manner as described above, a plasmid comprising the gene coding the mouse H chain V region derived from the hybridoma MABL-1 was prepared from the purified DNA fragment and designated as pGEM-M1H.

A plasmid comprising the gene coding the mouse kappa type L chain V region derived from the hybridoma MABL-2 was prepared from the purified DNA fragment and designated as pGEM-M2L.

A plasmid comprising the gene coding the mouse H chain V region derived from the hybridoma MABL-2 was prepared from the purified DNA fragment and designated as pGEM-M2H.

### Example 2 (DNA Sequencing)

The nucleotide sequence of the cDNA encoding region in the aforementioned plasmids was determined using Auto DNA Sequencer (Applied Biosystem) and ABI PRISM Dye Terminator Cycle Sequencing Ready Reaction Kit (Applied Biosystem) according to the manufacturer's protocol.

The nucleotide sequence of the gene coding the L chain V region from the mouse MABL-1 antibody, which is included in the plasmid pGEM-M1L, is shown in SEQ ID No.: 5.

The nucleotide sequence of the gene coding the H chain V region from the mouse MABL-1 antibody, which is included in the plasmid pGEM-M1H, is shown in SEQ ID No.: 6.

The nucleotide sequence of the gene coding the L chain V region from the mouse MABL-2 antibody, which is included in the plasmid pGEM-M2L, is shown in SEQ ID No.: 7.

The nucleotide sequence of the gene coding the H chain V region from the mouse MABL-2 antibody, which is included in the plasmid pGEM-M2H, is shown in SEQ ID No.: 8.

### Example 3 (Determination of CDR)

The V regions of L and H chains generally have a similarity in their structures and each four framework regions therein are linked by three hypervariable regions, i.e., complementatarity determining regions (CDR). An amino acid sequence of the framework is relatively well conserved, while an amino acid sequence of CDR has extremely high variation (Kabat, E.A., et al., "Sequences of Proteins of Immunological Interest", US Dept. Health and Human Services, 1983).

On the basis of these facts, the amino acid sequences of the variable regions from the mouse monoclonal antibodies to human IAP were applied to the database of amino acid sequences of the antibodies made by Kabat et al. and the homology thereof was investigated to determine the CDR. The results are shown in Table 1.

**Table 1**

| Plasmid | SEQ ID No. | CDR(1) | CDR(2) | CDR(3) |
|---|---|---|---|---|
| pGEM-M1L | 5 | 43-58 | 74-80 | 113-121 |
| pGEM-M1H | 6 | 50-54 | 69-85 | 118-125 |
| PGEM-M2L | 7 | 43-58 | 74-80 | 113-121 |
| pGEM-M1H | 8 | 50-54 | 69-85 | 118-125 |

### Example 4 (Identification of Cloned cDNA (Preparation of Chimera MABL-1 and MABL-2 Antibodies))

### 4.1 Preparation of a vector expressing chimera MABL-1 antibody

cDNA clones, pGEM-M1L and pGEM-M1H, encoding the V regions of the L chain and the H chain of the mouse MABL-1 antibody, respectively, were modified by the PCR method and introduced into the HEF expression vector (WO92/19759) in order to prepare a vector expressing chimera MABL-1 antibody.

A forward primer MLS (SEQ ID No.: 9) for the L chain V region and a forward primer MHS (SEQ ID No.: 10) for the H chain V region were designed to hybridize to a DNA encoding the beginning of the leader sequence of each V region and to contain the Kozak consensus sequence (J. Mol. Biol., 196, 947-950, 1987) and HindIII restriction enzyme site. A reverse primer MLAS (SEQ ID No.: 11) for the L chain V region and a reverse primer MHAS (SEQ ID No.: 12) for the H chain V region were designed to hybridize to a DNA encoding the end of the J region and to contain the splice donor sequence and BamHI restriction enzyme site.

100 µl of a PCR solution comprising 10 µl of 10 × PCR Buffer II, 2 mM MgCl₂, 0.16 mM dNTPs (dATP, dGTP, dCTP and dTTP), 5 units of DNA polymerase AmpliTaq Gold, 0.4 µM each of primers and 8 ng of the template DNA (pGEM-M1L or pGEM-M1H) was preheated at 94°C of the initial temperature for 9 minutes and then heated at 94°C for 1 minute, at 60°C for 1 minute and at 72°C for 1 minute in this order. This temperature cycle was repeated 35 times and then the reaction mixture was further heated at 72°C for 10 minutes.

The PCR product was purified using the QIAquick PCR Purification Kit (QIAGEN) and then digested with HindIII and BamHI. The product from the L chain V region was cloned into the HEF expression vector, HEF-κ and the product from the H chain V region was cloned into the HEF expression vector, HEF-γ. After DNA sequencing, plasmids containing a DNA fragment with a correct DNA sequence are designated as HEF-M1L and HEF-M1H, respectively.

### 4.2 Preparation of a vector expressing chimera MABL-2 antibody

Modification and cloning of cDNA were performed in the same manner as described in Example 4.1 except for amplifying pGEM-M2L and pGEM-M2H as template DNA instead of pGEM-M1L and pGEM-M1H. After DNA sequencing, plasmids containing a DNA fragment with a correct DNA sequence are designated as HEF-M2L and HEF-M2H, respectively.

### 4.3 Transfection to COS7 cell

The Expression of the aforementioned expression vectors was tested in COS7 cell to observe the transient expression of the chimera MABL-1 and MABL-2 antibodies.

### (1) Transfection with a gene coding the chimera MABL-1 antibody

COS7 cells were co-transformed with the HEF-M1L and HEF-M1H vectors by an electroporation using the Gene Pulser apparatus (BioRad). Each DNA (10 µg) and 0.8 ml of 1 × 10⁷ cells/ml in PBS were added to a cuvette and pulse was given at 1.5 kV, 25 µF of electric capacity.

After the restoration for 10 minutes at room temperature, the electroporated cells were transferred into DMEM culture media (GIBCO BRL) containing 10% γ-globulin free fetal bovine serum. After culturing for 72 hours, the cultured supernatant was collected and cell shard was removed by centrifugation to obtain the recovered supernatant.

### (2) Transfection with a gene coding the chimera MABL-2 antibody

The co-transfection to COS7 cells with the gene coding the chimera MABL-2 antibody was carried out in the same manner as described in Example 4.3-(1) except for using the HEF-M2L and HEF-M2H vectors instead of the HEF-M1L and HEF-M1H vectors to obtain the recovered supernatant.

### 4.4 Flow cytometry

Flow cytometry was performed using the aforementioned COS7 cells cultured supernatant in order to measure binding to the antigen. The cultured supernatant of the COS7 cells expressing the chimera MABL-1 antibody or the COS7 cells expressing the chimera MABL-2 antibody, or human IgG antibody (SIGMA) as a control was added to 4 × 10⁵ cells of mouse leukemia cell line L1210 expressing human IAP and incubated on ice. After washing, the FITC-labeled antihuman IgG antibody (Cappel) was added thereto. After incubating and washing, the fluorescence intensity thereof was measured using the FACScan apparatus (BECTON DICKINSON).

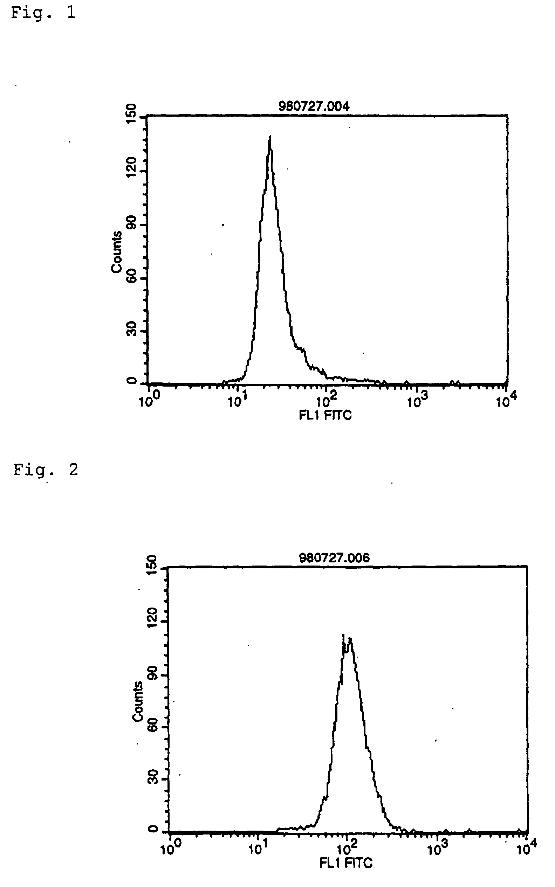

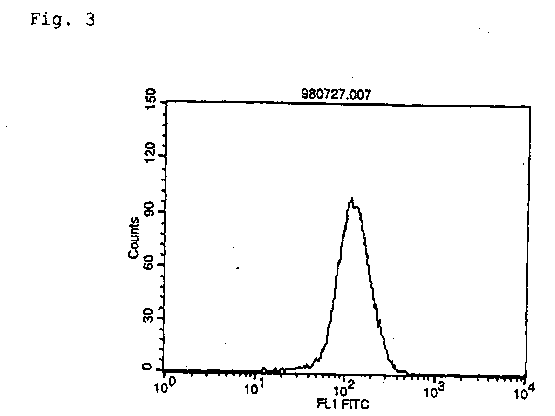

Since the chimera MABL-1 and MABL-2 antibodies were specifically bound to L1210 cells expressing human IAP, it is confirmed that these chimera antibodies have proper structures of the V regions of the mouse monoclonal MABL-1 and MABL-2 antibodies, respectively (Figures 1-3).

### Example 5 (Preparation of Single-chain Fv (scFv) of the Reconstructed MABL-1 and MABL-2 Antibodies)

### 5.1 Preparation of reconstructed single-chain Fv of MABL-1 antibody

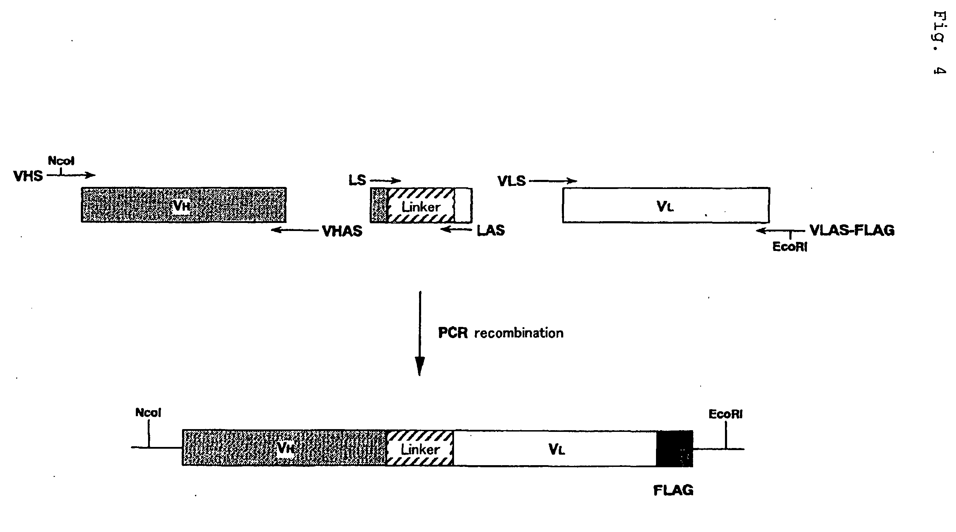

The reconstructed single-chain Fv of MABL-1 antibody was prepared as follows. The H chain V region and the L chain V of MABL-1 antibody, and a linker were respectively amplified by the PCR method and were connected to produce the single-chain Fv of MABL-1 antibody. The production method is illustrated in Figure 4. Six primers (A-E) were employed for the production of the single-chain Fv of MABL-1 antibody. Primers A, C and E have a sense sequence and primers B, D and F have an antisense sequence.

The forward primer VHS (Primer A, SEQ ID No.: 13) for the H chain V region was designed to hybridize to a DNA encoding the N-terminal of the H chain V region and to contain Nco I restriction enzyme recognition site. The reverse primer VHAS (Primer B, SEQ ID No.: 14) was designed to hybridize to a DNA coding the C-terminal of the H chain V region and to overlap with the linker.

The forward primer LS (Primer C, SEQ ID No.: 15) for the linker was designed to hybridize to a DNA encoding the N-terminal of the linker and to overlap with a DNA encoding the C-terminal of the H chain V region. The reverse primer LAS (Primer D, SEQ ID No.: 16) was designed to hybridize to a DNA encoding the C-terminal of the linker and to overlap with a DNA encoding the N-terminal of the L chain V region.

The forward primer VLS (Primer E, SEQ ID No.: 17) for the L chain V region was designed to hybridize to a DNA encoding the C-terminal of the linker and to overlap with a DNA encoding the N-terminal of the L chain V region. The reverse primer VLAS-FLAG (Primer F, SEQ ID No.: 18) was designed to hybridize to a DNA encoding the C-terminal of the L chain V region and to have a sequence coding the FLAG peptide, two stop codons and EcoRI restriction enzyme recognition site.

In the first PCR step, three reactions, A-B, C-D and E-F, were carried out and PCR products thereof were purified. Three PCR products obtained from the first PCR step were assembled by their complementarity. Then, the primers A and F were added to them and a full length DNA encoding the single-chain Fv of MABL-1 antibody was amplified (Second PCR). In the first PCR, a plasmid pGEM-M1H coding the H chain V region of MABL-1 antibody (see Example 2), a plasmid pSC-DP1 which comprises a DNA sequence coding a linker region comprising: Gly Gly Gly Gly Ser Gly Gly Gly Gly Ser Gly Gly Gly Gly Ser (SEQ ID No.: 19) (Huston, J.S., et al., Proc. Natl. Acad. Sci. USA, 85, 5879-5883, 1988) and the plasmid pGEM-M1L coding the L chain V region of MABL-1 antibody (see Example 2) were employed as a template, respectively.

50 µl of the solution for the first PCR step comprises 5 µl of 10 × PCR Buffer II, 2 mM MgCl₂, 0.16 mM dNTPs, 2.5 units of DNA polymerase, AmpliTaq Gold (PERKIN ELMER, respectively), 0.4 µM of each primers and 5 ng of each template DNA. The PCR solution was preheated at 94°C of the initial temperature for 9 minutes and then heated at 94°C for 1 minute, at 65°C for 1 minute and at 72°C for 1 minute and 20 seconds in this order. This temperature cycle was repeated 35 times and then the reaction mixture was further heated at 72°C for 7 minutes.

The PCR products A-B (371bp), C-D (63bp) and E-F (384bp) were purified using the QIAquick PCR Purification Kit (QIAGEN) and were assembled for the second PCR. In the second PCR, 98 µl of a PCR mixture comprising 120 ng of the first PCR product A-B, 20 ng of the PCR product C-D and 120 ng of the PCR product E-F, 10 µl of 10 × PCR Buffer II, 2mM MgCl₂, 0.16 mM dNTPs, 5 units of DNA polymerase AmpliTaq Gold (PERKIN ELMER) was preheated at 94°C of the initial temperature for 8 minutes and then heated at 94°C for 2 minutes, at 65°C for 2 minutes and at 72°C for 2 minutes in this order. This temperature cycle was repeated twice and then 0.4 µM each of primers A and F were added into the reaction, respectively. The mixture was preheated at 94°C of the initial temperature for 9 minutes and then heated at 94°C for 1 minute, at 60°C for 1 minute and at 72°C for 1 minute and 20 seconds in this order. This temperature cycle was repeated 35 times and then the reaction mixture was further heated at 72°C for 7 minutes.

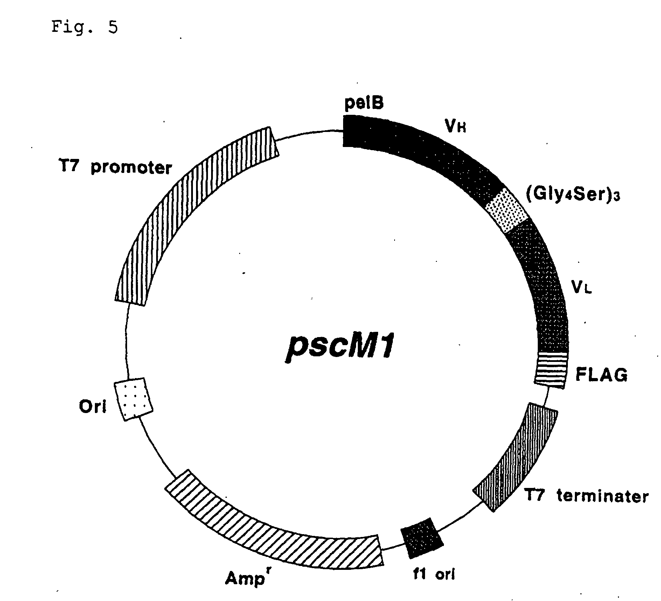

A DNA fragment of 843 bp produced by the second PCR was purified and digested by NcoI and EcoRI. The resultant DNA fragment was cloned into pSCFVT7 vector. The expression vector pSCFVT7 contains a pelB signal sequence suitable for E. coli periplasmic expression system (Lei, S.P., et al., J. Bacteriology, 169, 4379-4383, 1987). After the DNA sequencing, a plasmid containing a DNA fragment encoding a correct amino acid sequence of the single-chain Fv of MABL-1 antibody is designated as "pscM1" (see Figure 5). A nucleotide sequence and an amino acid sequence of the single-chain Fv of MABL-1 antibody contained in the plasmid pscM1 are shown in SEQ ID No.: 20.

pscM1 vector was modified by the PCR method in order to prepare a vector expressing the single-chain Fv of MABL-1 antibody in mammal cells. The resultant DNA fragment was introduced into pCHO1 expression vector. This expression vector, pCHO1, is constructed in the manner that an antibody gene is excluded from DHFR-△E-rvH-PM1-f (WO92/19759) by digesting with EcoRI and SmaI and the ECORI-NotI-BamHI Adapter (Takara shuzo) is linked thereto.

As a forward primer for PCR, Sal-VHS primer shown in SEQ ID No.: 21 was designed to hybridize to a DNA encoding the N-terminal of the H chain V region and to contain SalI restriction enzyme recognition site. As a reverse primer for PCR, FRH1-anti primer shown in SEQ ID No.: 22 was designed to hybridize to a DNA encoding the end of the first framework sequence.

100 µl of the solution comprising 10 µl of 10 × PCR Buffer II, 2 mM MgCl₂, 0.16 mM dNTPs, 5 units of the DNA polymerase, AmpliTaq Gold, 0.4 µl M of each primer and 8 ng of a template DNA (pscM1) was preheated at 95°C of the initial temperature for 9 minutes and then heated at 95°C for 1 minute, at 60°C for 1 minute and at 72°C for 1 minute and 20 seconds in this order. This temperature cycle was repeated 35 times and then the reaction mixture was further heated at 72°C for 7 minutes.

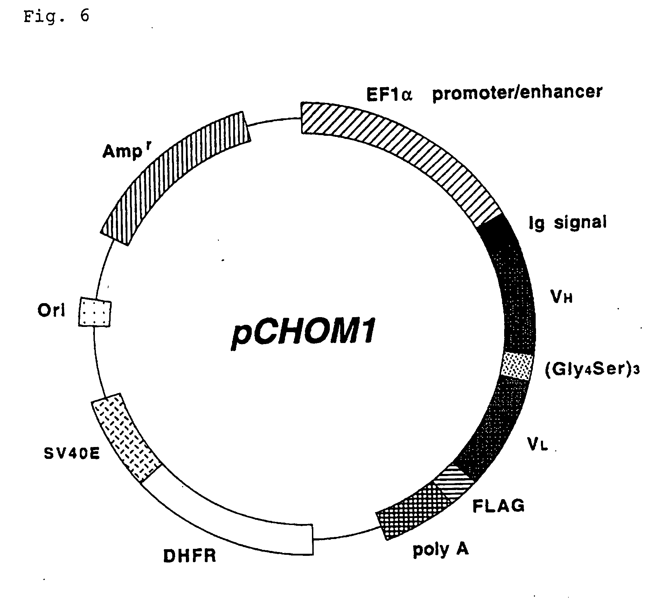

The PCR product was purified using the QIAquick PCR Purification Kit (QIAGEN) and digested by SalI and MboII to obtain a DNA fragment encoding the N-terminal of the single-chain Fv of MABL-1 antibody. The pscM1 vector was digested by MboII and EcoRI to obtain a DNA fragment encoding the C-terminal of the single-chain Fv of MABL-1 antibody. The SalI-MboII DNA fragment and the MboII-EcoRI DNA fragment were cloned into pCHO1-Igs vector. After DNA sequencing, a plasmid comprising the desired DNA sequence is designated as "pCHOM1" (see Figure 6). The expression vector, pCHO1-Igs, contains a mouse IgG1 signal sequence suitable for the secretion-expression system in mammal cells (Nature, 322, 323-327, 1988). A nucleotide sequence and an amino acid sequence of the single-chain Fv of MABL-1 antibody contained in the plasmid pCHOM1 are shown in SEQ ID No.: 23.

### 5.2 Preparation of reconstructed single-chain Fv of MABL-2 antibody

The reconstructed single-chain Fv of MABL-2 antibody was prepared in accordance with the aforementioned Example 5.1. Employed in the first PCR step were a plasmid pGEM-M2H coding the H chain V region of MABL-2 (see Example 2) instead of pGEM-M1H and a plasmid pGEM-M2L coding the L chain V region of MABL-2 (see Example 2) instead of pGEM-M1L, to obtain a plasmid pscM2 which comprises a DNA fragment encoding the desired amino acid sequence of the single-chain Fv of MABL-2 antibody. A nucleotide sequence and an amino acid sequence of the single-chain Fv of MABL-2 antibody contained in the plasmid pscM2 are shown in SEQ ID No.: 24.

pscM2 vector was modified by the PCR method to prepare a vector, pCHOM2, for the expression in mammal cells which contains a DNA fragment encoding the desired amino acid sequence of the single-chain Fv of MABL-2 antibody. A nucleotide sequence and an amino acid sequence of the single-chain Fv of MABL-2 antibody contained in the pCHOM2 plasmid are shown in SEQ ID No.: 25.

### 5.3 Transfection to COS7 cells

The pCHOM2 vector was tested in COS7 cells in order to observe the transient expression of the reconstructed single-chain Fv of MABL-2 antibody.

The COS7 cells were transformed with the pCHOM2 vector by electroporation using the Gene Pulser apparatus (BioRad). The DNA (10 µg) and 0.8 ml of 1 × 10⁷ cells/ml in PBS were added to a cuvette and pulse was given at 1.5 kV, 25 µF of electric capacity.

After the restoration for 10 minutes at room temperature, the electroporated cells were transferred into the IMDM culture media (GIBCO BRL) containing 10% fetal bovine serum. After culturing for 72 hours, the cultured supernatant was collected and cell shard was removed by centrifugation to obtain the withdrawn supernatant.

### 5.4 Detection of the reconstructed single-chain Fv of MABL-2 antibody in the cultured supernatant of COS7 cell

The existence of the single-chain Fv of MABL-2 antibody in the cultured supernatant of COS7 cells which had been transfected with the pCHOM2 vector was confirmed by the Western Blotting method.

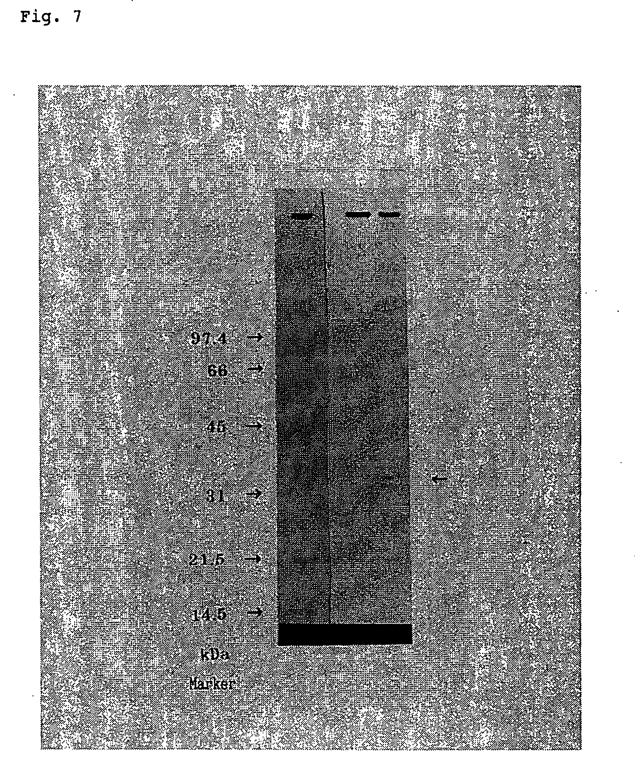

The cultured supernatant of COS7 cells transfected with the pCHOM2 vector and the cultured supernatant of COS7 cells transfected with the pCHO1 as a control were subjected to SDS electrophoresis and transferred to REINFORCED NC membrane (Schleicher & Schuell). The membrane was blocked with 5% skim milk (Morinaga Nyu-gyo) and washed with 0.05% Tween 20-PBS. Subsequently, an anti-FLAG antibody (SIGMA) was added thereto. The membrane was incubated at room temperature and washed and then the alkaline phosphatase-conjugated mouse IgG antibody (Zymed) was added. After the incubation and washing at room temperature, the substrate solution (Kirkegaard Perry Laboratories) was added and chromogenized (Figure 7).

The FLAG-peptide specific protein was detected only in the cultured supernatant of the pCHO1 vector-introduced COS7 cells and thus it is confirmed that the single-chain Fv of MABL-2 antibody was secreted in this cultured supernatant.

### 5.5 Flow cytometry

Flow cytometry was performed using the aforementioned COS7 cells cultured supernatants in order to measure the binding to the antigen. The cultured supernatant of the COS7 cells expressing the single-chain Fv of MABL-2 antibody or the cultured supernatant of COS7 cells transformed with pCHO1 as a control was added to 2 × 10⁵ cells of the mouse leukemia cell line L1210 expressing human Integrin Associated Protein (IAP) or the cell line L1210 transformed with pCOS1 as a control. After incubation on ice and washing, the mouse anti-FLAG antibody (SIGMA) was added and then the cells were incubated and washed. Then, the FITC labeled anti-mouse IgG antibody (BECTON DICKINSON) was added thereto and the cells were incubated and washed again. Subsequently, the fluorescence intensity was measured using the FACScan apparatus (BECTON DICKINSON).

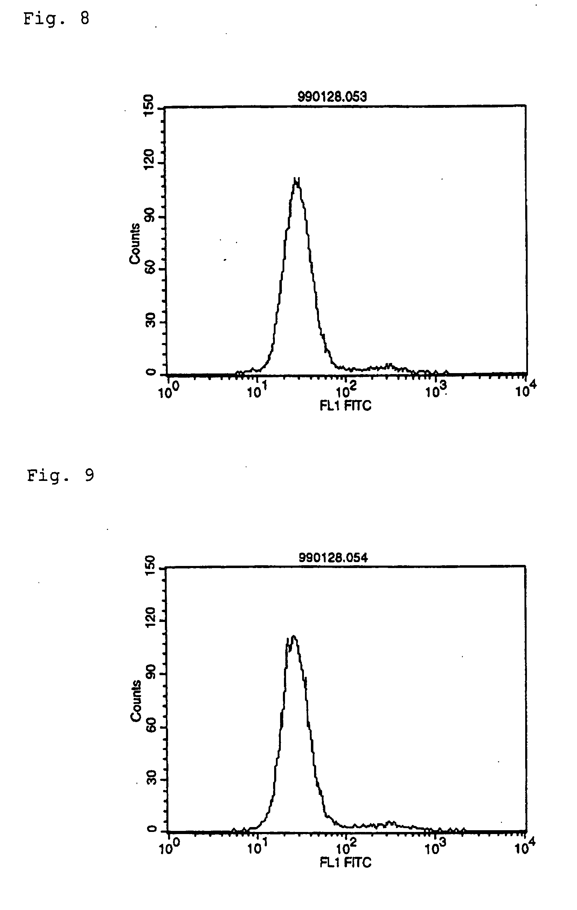

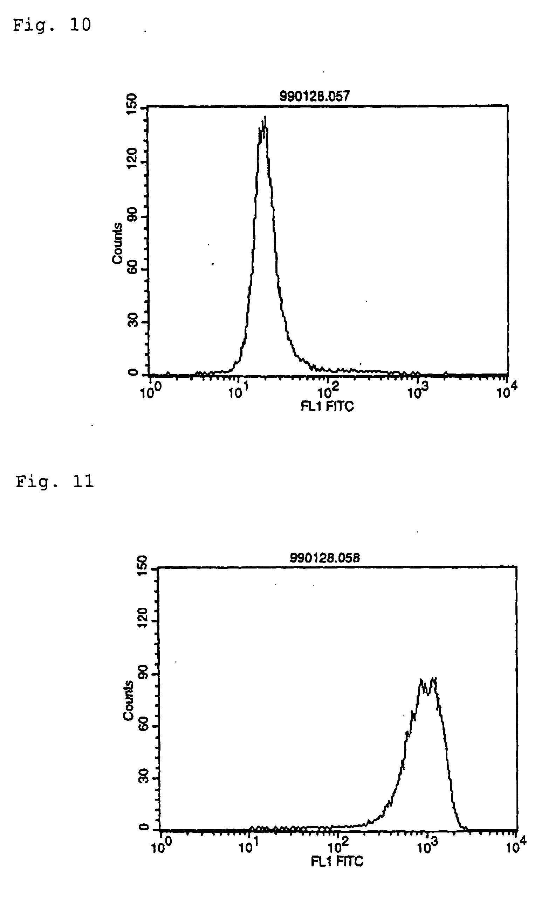

Since the single-chain Fv of MABL-2 antibody was specifically bound to L1210 cells expressing human IAP, it is confirmed that the single-chain Fv of MABL-2 antibody has an affinity to human Integrin Associated Protein (IAP) (see Figures 8-11).

### 5.6 Competitive ELISA

The binding activity of the single-chain Fv of MABL-2 antibody was measured using the inhibiting activity against the binding of mouse monoclonal antibodies to the antigen as an index.

The anti-FLAG antibody adjusted to 1 µg/ml was added to each well on 96-well plate and incubated at 37°C for 2 hours. After washing, blocking was performed with 1% BSA-PBS. After the incubation at room temperature and washing, the cultured supernatant of COS7 cells into which the soluble human IAP antigen gene (SEQ ID No.: 26) had been introduced was diluted twice with PBS and added to each well. After incubation at room temperature and washing, a mixture of 50 µl of the biotinized MABL-2 antibody adjusted to 100 ng/ml and 50 µl of sequentially diluted supernatant of the COS7 cells expressing the single-chain Fv of MABL-2 antibody was added into each well, incubated at room temperature and washed. Then, the alkaline phosphatase-conjugated streptoavidin (Zymed) was added into each well. After incubation at room temperature and washing, the substrate solution (SIGMA) was added and an absorbance at 405 nm of the reaction mixture in each well was measured.

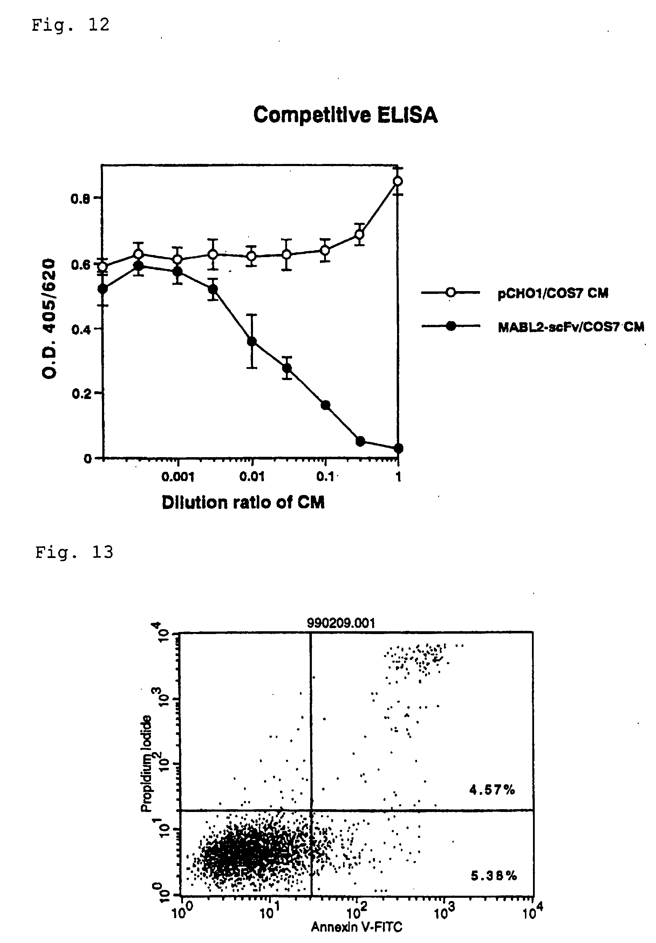

The results revealed that the single-chain Fv of MABL-2 antibody (MABL2-scFv) remarkably inhibited the binding of the mouse MABL-2 antibody to human IAP antigen dependent on the concentration thereof in comparison with the cultured supernatant of the PCHO1-introduced COS7 cells as a control (Figure 12). Accordingly, it is suggested that the single-chain Fv of MABL-2 antibody has a correct construction of each of the V regions from the mouse monoclonal antibody MABL-2.

### 5.7 Apoptosis-inducing Effect in vitro

An apoptosis-inducing action of the single-chain Fv of MABL-2 antibody was examined by Annexin-V staining (Boehringer Mannheim) using the L1210 cells transfected with human IAP gene, the L1210 cells transfected with the pCOS1 vector as a control and CCRF-CEM cells.

To each 1 × 10⁵ cells of the above cells was added a cultured supernatant of the COS7 cells expressing the single-chain Fv of MABL-2 antibody or a cultured supernatant of COS7 cells transfected with the pCHO1 vector as a control at 50% final concentration and the mixtures were cultured for 24 hours. Then, the Annexin-V staining was performed and the fluorescence intensity was measured using the FACScan apparatus (BECTON DICKINSON).

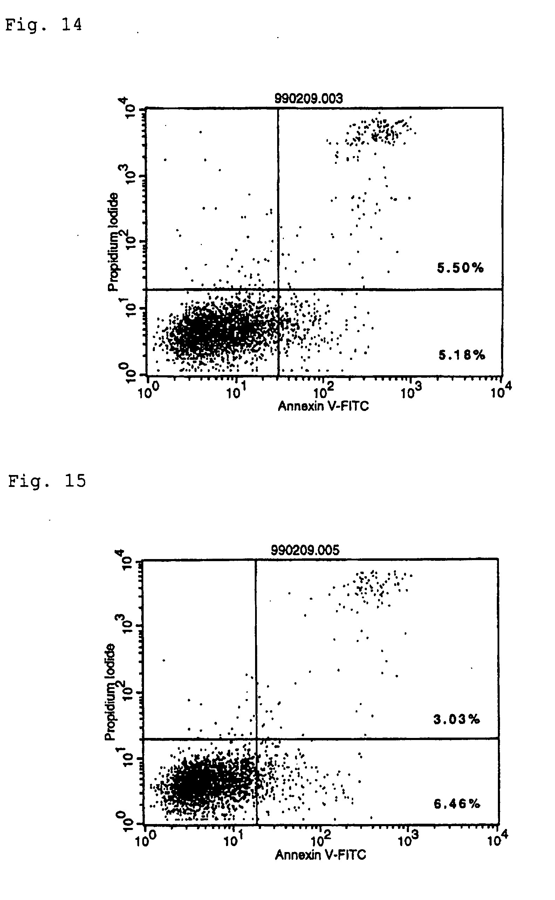

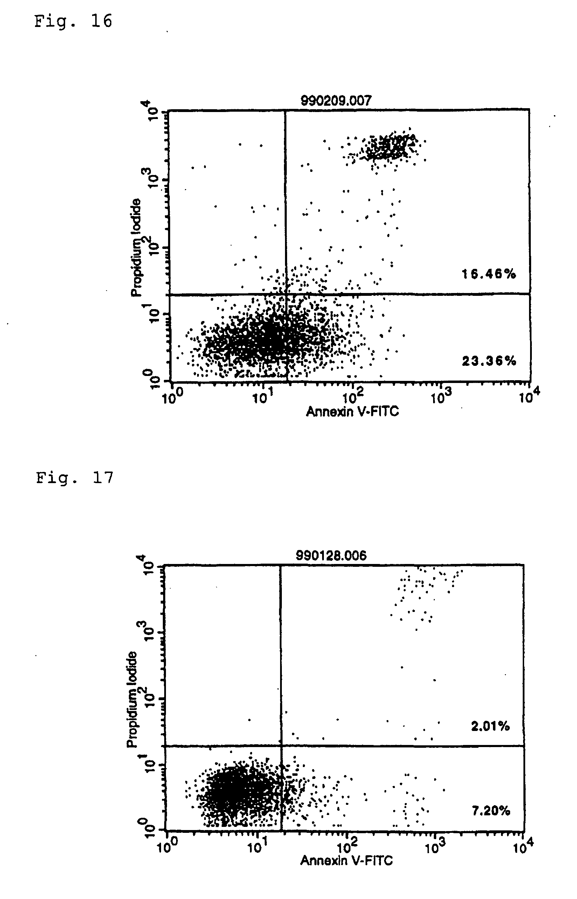

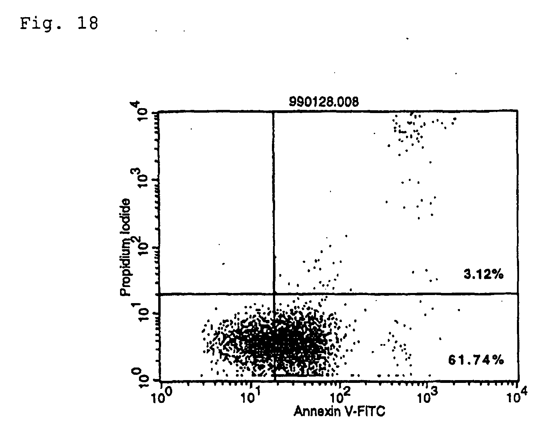

Results of the Annexin-V staining are shown in Figures 13-18, respectively. Dots in the left-lower region represent living cells and dots in the right-lower regions represent cells at the early stage of apoptosis and dots in the right-upper region represents cells at the late stage of apoptosis. The results show that the single-chain Fv of MABL-2 antibody (MABL2-scFv) remarkably induced human IAP specific cell death of L1210 cells (Figures 13-16) and that the single-chain Fv also induced cell death of CCRF-CEM cells in comparison with the control (Figures 17-18).

### INDUSTRIAL APPLICABILITY

According to this invention, novel single-chain Fvs capable of inducing apoptosis of nucleated blood cells with Integrin Associated Protein (IAP) have the aforementioned amino acid sequences. The single-chain Fvs specifically recognize nucleated blood cells with human IAP and are capable of inducing apoptosis of the cells. Therefore, the single-chain Fvs of the invention are useful as a therapeutic agent for blood dyscrasia such as myeloid leukemia and lymphoblastic leukemia.

## Claims

1. A polypeptide which is reconstructed with variable regions of the monoclonal antibodies capable of inducing apoptosis of nucleated blood cells having Integrin Associated Protein (IAP).

2. A DNA encoding a polypeptide of claim 1.

3. A single-chain Fv capable of inducing apoptosis of nucleated blood cells having Integrin Associated Protein (IAP).

4. An L chain V region comprising an amino acid sequence selected from
a) an amino acid sequence of SEQ ID No.: 5: 
Met Lys Leu Pro Val Arg Leu Leu Val Leu Met Phe Trp Ile Pro

Ala Ser Ser Ser Asp Val Val Met Thr Gln Thr Pro Leu Ser Leu

Pro Val Ser Leu Gly Asp Gln Ala Ser Ile Ser Cys Arg Ser Ser

Gln Ser Leu Leu His Ser Lys Gly Asn Thr Tyr Leu Gln Trp Tyr

Leu Gln Lys Pro Gly Gln Ser Pro Lys Leu Leu Ile Tyr Lys Val

Ser Asn Arg Phe Ser Gly Val Pro Asp Arg Phe Ser Gly Ser Gly

Ser Gly Thr Asp Phe Thr Leu Lys Ile Ser Arg Val Glu Ala Glu

Asp Leu Gly Val Tyr Phe Cys Ser Gln Ser Thr His Val Pro Tyr

Thr Ser Gly Gly Gly Thr Lys Leu Glu Ile Lys;
b) an amino acid sequence of SEQ ID No.: 7: 
Met Lys Leu Pro Val Arg Leu Leu Val Leu Met Phe Trp Ile Pro

Gly Ser Ser Ser Asp Val Val Met Thr Gln Ser Pro Leu Ser Leu

Pro Val Ser Leu Gly Asp Gln Ala Ser Ile Ser Cys Arg Ser Ser

Gln Ser Leu Val His Ser Asn Gly Lys Thr Tyr Leu His Trp Tyr

Leu Gln Lys Pro Gly Gln Ser Pro Lys Leu Leu Ile Tyr Lys Val
 
Ser Asn Arg Phe Ser Gly Val Pro Asp Arg Phe Ser Gly Ser Gly
Ser Val Thr Asp Phe Thr Leu Met Ile Ser Arg Val Glu Ala Glu
Asp Leu Gly Val Tyr Phe Cys Ser Gln Ser Thr His Val Pro Tyr
Thr Phe Gly Gly Gly Thr Lys Leu Glu Ile Lys;
 or
c) an amino acid sequence in which one or some amino acids are deleted, replaced or added into the amino acid sequence of a) or b).

5. An H chain V region comprising an amino acid sequence selected from
a) an amino acid sequence of SEQ ID No.: 6: 
Met Glu Trp Ser Trp Ile Phe Leu Phe Leu Leu Ser Gly Thr Ala
Gly Val His Ser Gln Val Gln Leu Gln Gln Ser Gly Pro Asp Leu
Val Lys Pro Gly Ala Ser Val Lys Met Ser Cys Lys Ala Ser Gly
Tyr Thr Phe Val Asn His Val Met His Trp Val Lys Gln Lys Pro
Gly Gln Gly Leu Glu Trp Ile Gly Tyr Ile Tyr Pro Tyr Asn Asp
Gly Thr Lys Tyr Asn Glu Lys Phe Lys Gly Lys Ala Thr Leu Thr
Ser Glu Lys Ser Ser Ser Ala Ala Tyr Met Glu Leu Ser Ser Leu
Ala Ser Glu Asp Ser Ala Val Tyr Tyr Cys Ala Arg Gly Gly Tyr
Tyr Ser Tyr Asp Asp Trp Gly Gln Gly Thr Thr Leu Thr Val Ser
Ser;
b) an amino acid sequence of SEQ ID No.: 8: 
Met Glu Trp Ser Trp Ile Phe Leu Phe Leu Leu Ser Gly Thr Ala

Gly Val His Ser Gln Val Gln Leu Gln Gln Ser Gly Pro Glu Leu

Val Lys Pro Gly Ala Ser Val Lys Met Ser Cys Lys Ala Ser Gly

Tyr Thr Phe Ala Asn His Val Ile His Trp Val Lys Gln Lys Pro

Gly Gln Gly Leu Glu Trp Ile Gly Tyr Ile Tyr Pro Tyr Asn Asp

Gly Thr Lys Tyr Asn Glu Lys Phe Lys Asp Lys Ala Thr Leu Thr
 
Ser Asp Lys Ser Ser Thr Thr Ala Tyr Met Asp Leu Ser Ser Leu
Ala Ser Glu Asp Ser Ala Val Tyr Tyr Cys Ala Arg Gly Gly Tyr
Tyr Thr Tyr Asp Asp Trp Gly Gln Gly Thr Thr Leu Thr Val Ser
Ser;
 or
c) an amino acid sequence in which one or some amino acids are deleted, replaced or added into the amino acid sequence of a) or b).

6. A DNA encoding the L chain V region of claim 4.

7. A DNA encoding the H chain V region of claim 5.

8. The DNA of claim 6 wherein the DNA encoding the L chain V region is selected from
a) a DNA of SEQ ID No.: 5: 
atg aag ttg cct gtt agg ctg ttg gtg ctg atg ttc tgg att cct 45

gcg tcc agc agt gat gtt gtg atg acc caa act cca ctc tcc ctg 90

cct gtc agt ctt gga gat caa gcc tcc atc tct tgc aga tct agt 135

cag agc ctt cta cac agt aaa gga aac acc tat tta caa tgg tac 180

cta cag aag cca ggc cag tct cca aag ctc ctg atc tac aaa gtt 225

tcc aac cga ttt tct ggg gtc cca gac agg ttc agt ggc agt gga 270

tca ggg aca gat ttc aca ctc aag atc agc aga gtg gag gct gag 315

gat ctg gga gtt tat ttc tgc tct caa agt aca cat gtt ccg tac 360

acg tcc gga ggg ggg acc aag ctg gaa ata aaa c 394
b) a DNA of SEQ ID No.: 7: 
atg aag ttg cct gtt agg ctg ttg gtg ctg atg ttc tgg att cct  45

ggt tcc agc agt gat gtt gtg atg acc caa agt cca ctc tcc ctg  90

cct gtc agt ctt gga gat caa gcc tcc atc tct tgc aga tca agt 135

cag agc ctt gtg cac agt aat gga aag acc tat tta cat tgg tac 180

ctg cag aag cca ggc cag tct cca aaa ctc ctg atc tac aaa gtt 225
 
tcc aac cga ttt tct ggg gtc cca gac agg ttc agt ggc agt gga 270
tca gtg aca gat ttc aca ctc atg atc agc aga gtg gag gct gag 315
gat ctg gga gtt tat ttc tgc tct caa agt aca cat gtt ccg tac 360
acg ttc gga ggg ggg acc aag ctg gaa ata aaa c 394
 or
c) a DNA hybridizing to the DNA of a) or b) under the stringent condition.

9. The DNA of claim 7 wherein the DNA encoding the H chain V region is selected from
a) a DNA of SEQ ID No.: 6: 
atg gaa tgg agc tgg ata ttt ctc ttc ctc ctg tca gga act gca 45

ggt gtc cac tcc cag gtc cag ctg cag cag tct gga cct gac ctg 90

gta aag cct ggg gct tca gtg aag atg tcc tgc aag gct tct gga 135

tac acc ttc gtt aac cat gtt atg cac tgg gtg aag cag aag cca 180

ggg cag ggc ctt gag tgg att gga tat att tat cct tac aat gat 225

ggt act aag tac aat gag aag ttc aag ggc aag gcc aca ctg act 270

tca gag aaa tcc tcc agc gca gcc tac atg gag ctc agc agc ctg 315

gcc tct gag gac tct gcg gtc tac tac tgt gca aga ggg ggt tac 360

tat agt tac gac gac tgg ggc caa ggc acc act ctc aca gtc tcc 405

tca g 409
b) a DNA of SEQ ID No.: 8: 
atg gaa tgg agc tgg ata ttt ctc ttc ctc ctg tca gga act gca 45

ggt gtc cac tcc cag gtc cag ctg cag cag tct gga cct gaa ctg 90

gta aag cct ggg gct tca gtg aag atg tcc tgc aag gct tct gga 135

tac acc ttc gct aac cat gtt att cac tgg gtg aag cag aag cca 180

ggg cag ggc ctt gag tgg att gga tat att tat cct tac aat gat 225

ggt act aag tat aat gag aag ttc aag gac aag gcc act ctg act 270
 
tca gac aaa tcc tcc acc aca gcc tac atg gac ctc agc agc ctg 315

gcc tct gag gac tct gcg gtc tat tac tgt gca aga ggg ggt tac 360

tat act tac gac gac tgg ggc caa ggc acc act ctc aca gtc tcc 405

tca g 409
 or
c) a DNA hybridizing to the DNA of a) or b) under the stringent condition.

10. The single-chain Fv of claim 3 which is a humanized single-chain Fv capable of inducing apoptosis of cells having Integrin Associated Protein (IAP).

11. The L chain V region of claim 4 which is a humanized L chain V region.

12. The H chain V region of claim 5 which is a humanized H chain V region.

13. A DNA encoding the humanized single-chain Fv of claim 10.

14. A humanized monoclonal antibody or a fragment thereof which can be prepared from a humanized single-chain Fv capable of inducing apoptosis of cells having Integrin Associated Protein (IAP).

15. A DNA encoding the humanized monoclonal antibody or the fragment thereof set forth in claim 14.

16. An animal cell which is capable of produing the single-chain Fv, the monoclonal antibody or the fragment thereof set forth in any one of claims 1, 3, 10 and 14.

17. A microorganism which is capable of produing the single-chain Fv, the monoclonal antibody or the fragment thereof set forth in any one of claims 1, 3, 10 and 14.

18. A therapeutic agent for blood dyscrasia comprising a substance capable of inducing apoptosis of cells having Integrin Associated Protein (IAP).

19. The therapeutic agent of claim 18 **characterized in that** the blood dyscrasia is leukemia.

20. The therapeutic agent of claim 18 **characterized in that** the substance is the single-chain Fv, the monoclonal antibody or the fragment thereof set forth in any one of claims 1, 3, 10 and 14.
